# EUROPÄISCHE PATENTSCHRIFT

(11) **EP 1 244 434 B1**
(45) Veröffentlichungstag und Bekanntmachung des Hinweises auf die Patenterteilung: **17.03.2004**
(21) Anmeldenummer: 00985241.9
(22) Anmeldetag: 22.12.2000
(51) Int. Cl.: A61K 31/00

(54) **VERWENDUNG VON CHEMOTHERAPEUTIKA ZUR TOPISCHEN BEHANDLUNG**
USE OF CHEMOTHERAPEUTIC AGENTS
UTILISATION D'AGENTS CHIMIOTHERAPEUTIQUES

(30) Priorität: 22.12.1999 DE 19962470
(43) Veröffentlichungstag der Anmeldung: 02.10.2002
(60) Teilanmeldung: 03028047.3
(73) Patentinhaber: Schulz & Schlimbach GbR, 51379 Leverkusen (DE)
(72) Erfinder: SCHULZ, Hans-Herrmann, 51067 Köln (DE); SCHLIMBACH, Günther, 51469 Bergisch Gladbach (DE)
(74) Vertreter: Kutzenberger, Helga, Dr.
(86) Internationale Anmeldenummer: PCT/EP2000/013155
(87) Internationale Veröffentlichungsnummer: WO 2001/045679

(56) Entgegenhaltungen:
- WO-A-00/18404
- WO-A-99/07706
- US-A- 5 478 829
- US-A- 5 693 337
- US-A- 6 017 912
- S. T. BOYCE ET. AL.: "Cytotoxicity Testing of Topical Antimicrobial Agents on Human Keratinocytes and Fibroblasts for Cultured Skin Grafts." JOURNAL OF BURN CARE AND REHABILITATION, Bd. 16, März 1995 (1995-03), Seiten 97-103, XP001036942

## Beschreibung

Die vorliegende Erfindung betrifft die Verwendung von bestimmten Derivaten der Chinoloncarbonsäure oder Naphthyridoncarbonsäure und/oder 4H-Oxochinolizinen der allgemeinen Formel II zur Herstellung eines Arzneimittels zur topischen und/oder lokalen Behandlung und/oder Prophylaxe von bakteriell verursachten Erkrankungen im Mundbereich und/oder Zahnbereich und/oder Kieferbereich, zur perioperativer Prophylaxe oder zur topischen und/oder lokalen Behandlung des diabetischen Fuß-Syndroms bei Mensch oder Tier.

Bakterien können die Ursache einer Vielzahl von Erkrankungen sein und zudem die Wundheilung beeinträchtigen. Im Mundbereich wird beispielsweise die Zahnfäule (Karies) durch mundspezifische Keime hervorgerufen. Mundbakterien setzen Kohlenhydrate der Nahrung zu Säuren um, die den Zahnschmelz (Enamelum) und Dentin (Dentinum) auflösen können. Ist die Schmelzoberfläche eingebrochen, dringen die Bakterien weiter in das darunter liegende Dentin vor. In den radiär verlaufenden Dentinkanälchen liegen Fortsätze der Pulpa, so dass es im weiteren Verlauf zu einer partiellen oder totalen Infektion und damit Entzündung der Pulpa kommt. Eine Pulpaentzündung bewirkt einen verstärkten Blutandrang. Da die Pulpa in der starren Pulpenkammer liegt, kann sie sich nicht ausdehnen, es kommt zu Schmerzen. Erfolgt keine Behandlung, ist die weitere Folge ein Absterben des Pulpengewebes (Nekrose) und ein bakterieller Zerfall (Gangrän). Werden die gangränösen Massen nicht entfernt, sind Entzündungen ausserhalb der Wurzelspitze die Folge. Granulome, Zysten, Fistelbildung oder Abzesse können sich entwickeln. Durch Gasentwicklung treten auch jetzt verstärkt Schmerzen auf.

Auch an der Entzündung des Zahnhalteapparates (Parodontium) sind Bakterien beteiligt. Der Zahnhalteapparat besteht aus dem Zahnfleisch, dem Ringband, der Wurzelhaut und und den dazwischen liegenden Sharpey'schen Fasern. Eine Entzündung, die Parodontitis, kann einzelne Bereiche oder auch das gesamte Parodontium umfassen. Die Parodontitis ebenso wie Karies wird hervorgerufen durch Zahnbelag (Plaque) an den Rändern des Zahnfleisches, der sich durch Kalkeinlagerungen zum Zahnstein verfestigt In der Plaque lebende Bakterien bilden Stoffwechselprodukte, die eine Parodontopathie verursachen. Das Zahnfleisch bildet sich allmählich zurück, Wurzelhaut und Alveolarknochen beginnen sich aufzulösen. Im umgebenden Gewebe bilden sich Infektionsherde. Die Folgen sind die weitere Zerstörung der Knochen und eine Lockerung der Zähne. Schließlich fallen die Zähne aus. Schäden im Mundbereich können auch zu Erkrankungen anderer Körperorgane führen.

Zur Behandlung der Caries profunda werden sowohl bei freiliegender Pulpa als auch bei noch geschlossener Dentindecke Calciumhydroxid-Präparate oder Zinkoxid-Nelkenöl (Eugenol) eingesetzt. Eine infizierte und nekrotisierte Pulpa wird möglichst vollständig entfernt und das Pulpenkavum mit geeigneten Wurzelfüllmaterialien ausgefüllt. Liegt eine Gangrän der Pulpa vor, so ist ein Erfolg nur dann zu erwarten, wenn es gelingt, das verseuchte Wurzelkanalsystem und das kanalnahe Dentin zu desinfizieren. Das Hauptproblem besteht in der Unzugänglichkeit des bakteriell infizierten apikalen Deltas der Wurzelkanäle, das nur schwer zu reinigen ist, des weiteren in dem infizierten Kahalwandsystem, das mit erheblichem instrumentellen Aufwand entfernt werden muss. Eine Gangränbehandlung ist deshalb in der Regel eine Kompromisslösung und wird von einigen Schulen ganz abgelehnt. Als Kontraindikation für eine Gangränbehandlung gelten grundsätzlich mehrwurzlige Zähne und röntgenologisch sichtbare apikale Prozesse. In diesen Fällen ist die Extraktion des Zahnes indiziert. Parodontopathien werden durch die Entfernung subgingivaler Konkremente behandelt. Darüber hinaus werden auch Arzneimittel wie Kaustika, Antiphlogistika oder Vitamine für die Behandlung eingesetzt.

Für die Behandlung von Infektionen im Mundbereich setzt man antibakteriell wirksame Antibiotika und Chemotherapeutika ein, die jedoch nur nach kritischer Indikationsstellung verabreicht werden sollten. Die verwendeten Antibiotika wie Penicillin G oder Oralpenicilline sind die Mittel der ersten Wahl. Alternativ kommen Erythromycin, Lincomycin, Clindamycin, eventuell auch Sulfonamide in Betracht. Bei Mischinfektionen mit gramnegativen Keimen kommen Breitspektrum-Penicilline wie Ampicillin als Mittel der ersten Wahl in Betracht, die gegebenenfalls durch Tetracycline ersetzt werden können. Die Antibiotika und Chemotherapeutika werden immer systemisch gegeben. Es gilt die herrschende Lehrmeinung, dass zur systemischen Chemotherapie verwendete Mittel nicht als Lokalantibiotika eingesetzt werden sollen.

Den heutigen Einsatz von antimikrobiellen Substanzen in der Zahnmedizin beschreiben B. M. Owens und N. J. Schuman (Journal of Clinical Pediatric Dentistry, 1994 (Winter), 18, 129-134; zitiert in Medline, AN 94331337). Demnach werden zwei Kategorien von antimikrobiellen Wirkstoffen unterschieden, die natürlich vorkommenden Substanzen aus Pilzen (Penicilline und Cephalosporine), Bakterien und Actinomyceten (Aminoglycoside) und ihre Abkömmlinge, die Antibiotika genannt werden, sowie die Verbindungen synthetischen Ursprungs (Sulfanilamide und Chinolone), die Chemotherapeutika genannt werden. Die Gruppe der Antibiotika mit Bedeutung für die Zahnmedizin sind die Penicilline, Cephalosporine und Aminoglycoside sowie Erythromycin. Wegen ihrer guten Wirksamkeit, der niedrigen Kosten und der einfachen Anwendung sind diese Antibiotika die Mittel der Wahl für viele, wenn nicht sogar für die meisten odontogenen Infektionen. Die Substanzen synthetischen Ursprungs haben einen geringeren Wert für die Zahnmedizin. Es gilt die herrschende Lehrmeinung, dass sie häufig durch hohe Kosten, einen Mangel an Wirksamkeit und Toxizität für den Patienten gekennzeichnet sind.

In In-vitro-Studien wurde die antimikrobielle Empfindlichkeit von Keimen, die eine progressive Parodontitis oder odontogene Abzesse hervorrufen, von S. Eick et al. (Int. J. Antimicrob. Agents, 1999, 12, 41-46) gegenüber modernen Antibiotika und Chemotherapeutika, nämlich Penicillin, Amoxicillin, Cefoxitin, Clindamycin, Doxycyclin, Metronidazol und Ciprofloxacin untersucht. Als Ergebnis wurde Clindamycin für antimikrobielle Behandlungen empfohlen.

Zur Behandlung von Patienten wurden Antibiotika in der Zahnmedizin sowohl systemisch als auch topisch eingesetzt. U. Wahlmann et al. (Int. J. Antimicrob. Agents, 1999, 12, 253-256) berichteten über die Effekte der systemischen Gabe von Cefuroxim zehn Minuten vor der Extraktion von Zähnen. Die mit Cefuroxim behandelte Gruppe hatte eine geringere Häufigkeit an Bakteriämien als die nicht behandelte.

K. Kosowska und P. B. Heczko (Med. Dosw. Mikrobiol., 1977, 29, 101-106; zitiert in Chemical Abstracts, CA 88:69315) berichteten über den topischen Einsatz von verschiedenen Antibiotika. Sie behandelten infizierte Zähne mit verschiedenen Antibiotika, nämlich Erythromycin, Neomycin, Chloramphenicol, Colistin, Nystatin oder Dexamethason. In etwa 55 % der Fälle wurde die aerobe Flora in den Wurzelkanälen eliminiert. Durch die Behandlung wurde jedoch die antibiotische Resistenz der Mikroorganismen, die nach Behandlung aus den Wurzelkanälen isoliert worden waren, erhöht. Die Resistenz von Staphylococcus epidermis gegenüber Erythromycin oder Methicillin wurde um das dreifache bzw. zweifache erhöht. Die meisten Staphylococcus aureus Stämme waren resistent gegenüber Erythromycin, Chloramphenicol und Penicillin.

Die Autoren der genannten Quellen kommen übereinstimmend zu dem Ergebnis, dass der topische Einsatz von Antibiotika und Chemotherapeutika, die als Lutschtabletten, Halstabletten, Pastillen, Styli, Kegel, Puder oder Salben verabreicht werden, wegen der häufig auftretenden Resistenzsteigerungen der Erreger und der hohen Sensibilisierungsquote nur eine sehr geringe Bedeutung hat. Auch in Wunden mit ausreichendem Abfluss nach außen ist der therapeutische Nutzen im allgemeinen gering. Die verwendeten Substanzen werden kaum oder gar nicht resorbiert, so dass auch keine tiefer in das Gewebe gehende Wirkung zu erwarten ist.

Demnach besteht nach dem heutigen Stand der Technik in der humanen und tierärztlichen Zahnmedizin ein hohes Bedürfnis nach Mitteln, die eine hohe Aktivität gegen die im Mund-, Zahn- und Kieferbereich oder in oralen Wunden auftretenden Keime aufweisen, die schnell und bakterizid wirksam sind, die lokal gut verträglich sind, die eine geringe Neigung zur Erzeugung antibiotischer Resistenz hervorrufen, die topisch und/oder lokal angewendet werden können und somit einfach zu verabreichen sind, die bei topischer/lokaler Gabe eine minimale systemische Belastung des Organismus bewirken, die eine gute Gewebegängigkeit aufweisen, und deren Anwendung den Erhalt der geschädigten Zähne gewährleistet.

Bakterien sind auch bei der Wundversorgung von Bedeutung. Wunden sind Defekte des Gewebes an der Körperoberfläche und können durch Verletzungen, Operationen, Infektionen oder pathophysiologische Prozesse hervorgerufen werden. Wunden sind unter anderem wegen möglicher Infektionen aufgrund eindringender krankheitserregender Bakterien gefährlich. Die bakterielle Besiedelung der Wunde kann den Prozess der Wundheilung verlangsamen oder verhindern oder zu weiteren Komplikationen wie Lymphangitis, Sepsis oder chronischen Infektionen führen.

Infizierte Wunden müssen eine antimikrobielle Behandlung zur Bekämpfung pathogener Keime erfahren. Außerdem muß die Wunde - ähnlich wie bei nekrotischen Wunden - einer Reinigung von Fremdkörpern und Zelltrümmern unterzogen werden, damit sie in die nächste Heilungsphase eintreten kann. Die Behandlung einer infizierten Wunde besteht üblicherweise in einem kombinierten systemischen und lokalen Ansatz. Dabei werden gegebenenfalls Antibiotika eingesetzt und ein geeigneter Verband angelegt, der selbst antibakteriell wirken kann.

Im Journal of Bum Care and Rehabilitation, Band 16, S. 97-103 (1995) wird die topische Behandlung von infizierten Verbrennungen mit Sparfloxacin beschrieben

In der WO-A-00/18404, die nicht vorveröffentlicht ist, wird die Verwendung von Grepafloxacin für die topische Beahndlung von opthalischen Infektionen und in US-A-5,693,337 die Verwendung von Ofloxacin in Augentropfen beschrieben.

In vielen Fällen lassen sich Wundinfektionen durch Gabe von systemisch wirksamen Antibiotika erfolgreich behandeln. Die systemische Gabe eines Antibiotikums führt jedoch zwangsläufig zu einer Belastung des gesamten Organismus. Dies kann in vielen Fällen kontraindiziert sein, beispielsweise bedingt durch die klinische Situation des Patienten, bei Grunderkrankungen des Patienten oder bei Vorliegen eines Allergisierungspotentials. In solchen Fällen ist eine topische und/oder lokale Applikation des Antibiotikums von Vorteil, was dadurch auch in einer höheren lokalen Gewebekonzentration vorliegen kann. Auch andere Faktoren wie bei bestimmten Krankenhausepidemiologien oder ökonomische Aspekte wie notwendige Aufwandmengen, Medikamentenpreise, Kosten durch Nebenwirkungen können für topische/lokale Applikationen sprechen. Der Einsatz topischer Antibiotika wird jedoch nicht generell empfohlen, da dies zu allergischen Reaktionen oder zur Ausbildung von antibiotikaresistenten Arten von Bakterien führen kann. Deshalb wird es als besonders wichtig angesehen, den Einsatz topischer Antibiotika zu limitieren.

Eine antibiotische Lokalbehandlung wird heute nur bei oberflächlichen Hautinfektionen angewandt, weil das Antibiotikum direkt auf die Erreger einwirken kann. Eine örtliche Anwendung bei tiefen Hautinfektionen ist erfolglos, weil die Antibiotika nicht durch die intakte Haut penetrieren. Heute werden bevorzugt drei Gruppen von Lokalantibiotika verwendet. Dies sind die Polypeptide wie Bacitracin, Tyrothricin, Colistin und Polymxyin B oder Aminoglycoside wie Neomycin, Kanamycin und Paromycin oder Mupirocin. Jedoch sollten Lokalantibiotika bei vorhandener lokaler bzw. systemischer Toxizität und der Gefahr einer sekundären Resistenzentwicklung der Bakterien nur mit großer Zurückhaltung angewendet werden. Bei den Lokaltherapien mit Gyrase-Hemmern sind neben konventionellen Applikationsformen wie Augentropfen, Ohrentropfen, Instillationslösungen, Puder und Wundsalben die Inkorporation in Kunststoffmaterialien als klinische Anwendungsmöglichkeiten vorgeschlagen worden (W. Stille, Fortschritte der antimikrobiellen und antineoplastischen Chemotherapie, Band 6-10, 1987, S. 1575 -1583).

Demnach besteht nach dem heutigen Stand der Technik auch in der Human- und Tiermedizin ein Bedürfnis nach topisch und/oder lokal anwendbaren antibiotisch wirksamen Mitteln, die eine hohe Aktivität gegen die in Wunden auftretenden Keime aufweisen, die schnell und bakterizid wirksam sind, die lokal gut verträglich sind, die eine geringe Neigung zur Erzeugung antibiotischer Resistenz hervorrufen, die bei topischer und/oder lokaler Anwendung einfach zu verabreichen sind, die bei topischer/lokaler Gabe eine minimale systemische Belastung des Organismus bewirken, die eine gute Gewebegängigkeit aufweisen, die auch zur Behandlung von tiefen Infektionen geeignet sind und die darüber hinaus den Prozess der Wundheilung beschleunigen.

Es wurde nun überraschenderweise gefunden, dass bestimmte Chemotherapeutika bei lokaler und/oder topischer Anwendung in vielfältiger Weise einen überaus positiven Effekt auf die Behandlung von bakteriell verursachten Erkrankungen im Mundbereich bei Mensch und Tier, wie beispielsweise Erkrankungen des Zahnes und des Zahnhalteapparates, und auf die Wundheilung haben. Arzneimittel, die diese Chemotherapeutika enthalten können in der Zahnmedizin erfolgreich eingesetzt werden gegen Keime, die im Weichgewebe und/oder im Hartgewebe angetroffen werden und dort zu Entzündungen führen. Die Arzneimittel sind allgemein geeignet zur lokalen Behandlung von endodontischen Krankheitsbildern wie Pulpitiden auf Grund von kariösen Erkrankungen, zur Prophylaxe bei Dentinwunden, zur topischen Behandlung des infizierten Wurzelkanals und des periapikalen Gewebes, ferner zur topischen Behandlung sowohl von Paradontopathien als auch von Schleimhaut-Knochenwunden mit gestörter Wundheilung zum Beispiel nach Zahnextraktion und von Weichteilinfektionen. Derartige Erkrankungen sind bakteriell verursachte Erkrankungen im Zahnhartgewebe wie zum Beispiel bei Infektionen im Kronen- und Wurzeldentin, im Dentin im Inneren des Wurzelkanals und im Wurzelzement im apikalen Bereich der Zahnwurzel. Ferner zählen dazu auch bakterielle Infektionen der Kiefer- und Alveolarknochen. Desweiteren sind darunter auch Infektionen in Weichgeweben wie in der Pulpa, im Parodontalgewebe, in der gingivalen Schleimhaut, in der Alveolarschleimhaut, in der Lippen- und Wangenschleimhaut, in der Gaumenschleimhaut und in der Zungenschleimhaut zu verstehen. Es wurde ferner überraschenderweise gefunden, dass die Verwendung dieser Chemotherapeutika in der Human- und Tiermedizin in vielfältiger Weise einen positiven Effekt auch bei der Behandlung und der Prophylaxe von Wunden haben. Dies wurde bei der Behandlung von verschiedenen Wundformen gezeigt, beispielsweise bei chirurgischen Infektionen wie postoperativen oder posttraumatischen Wundinfektionen, bei der periooperativen Prophylaxe, bei Handinfektionen, bei der postoperativen Sepsis, bei infizierten Ulzera und Gangränen, bei Hautinfektionen wie akuten und chronischen bakteriellen Hautinfektionen, sekundär infizierten Dermatosen oder Akne und Rosazea. Diese Aufzählungen sind beispielhaft und bedeuten keine Einschränkung auf die genannten Bereiche.

Diese Erkrankungen lassen sich erfindungsgemäß durch lokale und/oder topische Applikation von Chemotherapeutika behandeln.

Chemotherapeutika im erfindungsgemäßen Sinne sind die gemäß Anspruch 1 verwendeten Derivate der Chinoloncarbonsäure oder Naphthyridoncarbonsäure der allgemeinen Formel (I) 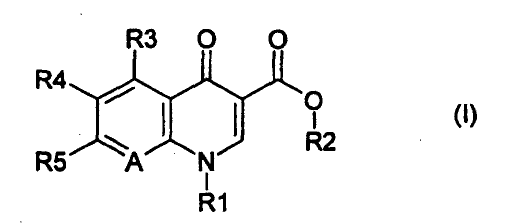 in welcher:
- A: für CH, C-Halogen, C-CH₃, C-CN, C-OCH₃, C-OCHF₂ oder N steht,
- R1: für C₁-C₅-Alkyl, C₁-C₅-Alkenyl, 2-Fluorethyl, Cycloalkyl, Bicycloalkyl, 2-Fluorcyclopropyl, 1-Oxetan-3-yl, Methylamino, gegebenenfalls substituiertes Phenyl oder Pyridyl steht, oder A und R1 gemeinsam die Gruppe C-O-CH₂-CH(CH₃)- bilden,
- R2: für Wasserstoff oder gegebenenfalls durch Hydroxy, Halogen oder Amino substituiertes C₁-C₃-Alkyl steht,
- R3: für Wasserstoff, Halogen, Methyl, Amino oder NH-NH₂ steht,
- R4: für Wasserstoff, Halogen oder Amino steht, und
- R5: für einen gegebenenfalls einfach oder mehrfach substituierten gesättigten oder wenigstens eine Doppelbindung aufweisenden mono-, bi- oder tricyclischen Alicyclus mit gegebenenfalls wenigstens einem Heteroatom im Ringsystem oder einen gegebenenfalls wenigstens ein Heteroatom aufweisenden aromatischen Mono-, Bi- oder Tricyclus steht,
und/oder 4H-4-Oxochinolizine der der allgemeinen Formel (II) 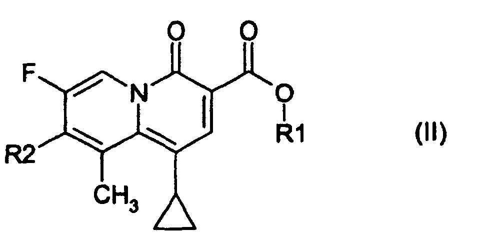 in welcher:
- R1: für Wasserstoff oder C₁-C₃-Alkyl steht und
- R2: für einen gegebenenfalls einfach oder mehrfach substituierten gesättigten oder wenigstens eine Doppelbindung aufweisenden mono-, bi- oder tricyclischen Alicyclus mit gegebenenfalls wenigstens einem Heteroatom im Ringsystem oder einen gegebenenfalls wenigstens ein Heteroatom aufweisenden aromatischen Mono-, Bi- oder Tricyclus steht,
und/oder ihre entsprechende Hydrate und/oder ihre entsprechenden physiologisch verträglichen Säureadditionssalze und/oder ggf. ihre entsprechenden physiologisch verträglichen Salze der zugrundeliegenden Carbonsäuren für Verbindungen der allgemeinen Formel (I), worin R2 für H steht, und/oder Verbindungen der allgemeinen Formel (II), worin R1 für H steht, und/oder entsprechende Enantiomere und/oder entsprechende Diastereomere und/oder entsprechende Racemate und/oder entsprechende Gemische aus wenigstens zwei der vorstehend genannten Verbindungen zur Herstellung eines Arzneimittels zu den anspruchsgemäßen Verwendungen.

Diese Verbindungen haben bei topischer und/oder lokaler Gabe eine förderliche Wirkung bei der Behandlung von bakteriell verursachten Erkrankungen im Mundbereich bei Mensch und Tier, insbesondere bei der Behandlung von Pulpitiden einschließlich der Infektionen des Wurzelkanals und des periapikalen Gewebes, Paradontopathien, odontogenen oder oralen Weichteilinfektionen, bei der Prophylaxe bei Dentinwunden und bei der Behandlung von Wundformen bei Mensch und Tier bei chirurgischen Infektionen wie postoperativen oder posttraumatischen Wundinfektionen, bei der periooperativen Prophylaxe, bei Handinfektionen, bei der postoperativen Sepsis, bei infizierten Ulzera und Gangränen, bei akuten und chronischen bakteriellen Hautinfektionen, sekundär infizierten Dermatosen oder Akne und Rosazea sowie allgemein zur Beschleunigung der Wundheilung bei Mensch und Tier.

Bevorzugt wird wenigstens eine dieser Verbindungen zur Herstellung eines pharmazeutischen Erzeugnisses, insbesondere Arzneimittels, zu anspruchsgemäßen, topischen und/oder lokalen Behandlungen von bakteriell verursachten Erkrankungen oder zur anspruchsgemäßen Beschleunigung der Wundheilung verwendet.

Bevorzugt ist die Verwendung der Verbindungen der Formel (I), in welcher
- A: für CH, CF, CCI, CBr, C-CH₃, C-CN, C-OCH₃, C-OCHF₂ oder N steht,
- R1: für Ethyl, 1,1-Dimethyl-ethyl, 1-Ethenyl, 1,1-Dimethyl-prop-2-ynyl, 2-Fluorethyl, Cyclopropyl, Bicyclo(1.1.1 )pent-1-yl, 2-Fluorcyclopropyl, 1-Oxetan-3-yl, Methylamino, 4-Fluorphenyl, 2,4-Difluorphenyl, 5-Amino-2,4-difluor-phenyl, 5-Fluor-pyridin-2-yl oder 6-Amino-3,5-diftuor-pyridin-2-yl steht oder A und R1 gemeinsam die Gruppe C-O-CH₂-CH(CH₃)-bilden, wobei das -CH(CH₃)-Glied dieser Gruppe an das Stickstoffatom des Heterozyklus gebunden ist,
- R2: für Wasserstoff, Methyl oder Ethyl steht.
- R3: für Wasserstoff, F, Cl, Br, Methyl, Amino oder NH-NH₂ steht,
- R4: für Wasserstoff, F oder Amino steht,
- R5: für gegebenenfalls einfach oder mehrfach substituiertes Cyclopropyl, Azetidinyl, Pyrrolidinyl, Piperidinyl, Piperazinyl, Morpholinyl, Phenyl, Pyrrolyl, Pyridyl oder Imidazolyl steht, wobei gegebenenfalls auch wenigstens zwei Substituenten miteinander verknüpft sein können, soweit sie den in Anspruch 1 aufgeführten Verbindungen entsprechen,
und/oder der Verbindungen der Formel (II), in welcher
- R1: für Wasserstoff steht und
- R2: für gegebenenfalls einfach oder mehrfach substituiertes Cyclopropyl, Azetidinyl, Pyrrolidinyl, Piperidinyl, Piperazinyl oder Morpholinyl steht, wobei gegebenenfalls auch wenigstens zwei Substituenten miteinander verknüpft sein können.

Besonders bevorzugt ist die Verwendung der Verbindungen der Formel (I), in welcher
- A: für CH, CF, CCI, C-CN, C-OCH₃ oder N steht,
- R1: für Cyclopropyl, 2-FluorcycJopropyl, 4-Fluorphenyl oder 2,4-Difluorphenyl steht oder A und R1 gemeinsam die Gruppe C-O-CH₂-CH(CH₃)- bilden, wobei das -CH(CH₃)-Glied dieser Gruppe an das Stickstoffatom des Heterozyklus gebunden ist,
- R2: für Wasserstoff steht,
- R3: für Wasserstoff oder Amino steht,
- R4: für Wasserstoff oder F steht,
- R5: für gegebenenfalls einfach oder mehrfach substituiertes Pyrrolidinyl, Piperidinyl, Piperazinyl oder Morpholinyl steht, wobei gegebenenfalls auch wenigstens zwei Substituenten miteinander verknüpft sein können, soweit sie den in Anspruch 1 aufgeführten Verbindungen entsprechen.

Ganz besonders bevorzugt ist die Verwendung der Verbindungen der Formel (I), in welcher
- A: für CH, CF, CCl, C-OCH₃ oder N steht,
- R1: für Cyclopropyl oder 2,4-Difluorphenyl steht oder A und R1 gemeinsam die Gruppe C-O-CH₂-CH(CH₃)- bilden, wobei das CH(CH₃)-Glied dieser Gruppe an das Stickstoffatom des Heterozyklus gebunden ist,
- R2: für Wasserstoff steht,
- R3: für Wasserstoff oder Amino steht,
- R4: für Wasserstoff oder F steht,
- R5: für gegebenenfalls mit Amino, Methyl, Aminomethyl und/oder Methoxyimino substituiertes Pyrrolidinyl, Piperidinyl, Piperazinyl, Morpholinyl, 3-Azabicyclo(3.1.0)hexyl oder für Piperidino-pyrrolidinyl steht, soweit sie den in Anspruch 1 aufgeführten Verbindungen entsprechen.

Unter bi- oder tricyclischen Alicyclen bzw. aromatischen Bi- oder Tricyclen gemäß R5 der allgemeinen Formel (I) und gemäß R2 der allgemeinen Formel (II) werden auch kondensierte Ringsysteme verstanden, die gegebenenfalls wenigstens eine Doppelbindung und/oder wenigstens ein Heteroatom im Ringsystem aufweisen können, soweit sie den in Anspruch 1 aufgeführten Verbindungen entsprechen.

Beispiele für Reste R5 in den Verbindungen der Formel (I) sind 3-Amino-pyrrolidin-1-yl, 3-Aminomethyl-4-methoxyimino-pyrrolidin-1-yl, 3-Methyl-1-piperazinyl, 4-Methylpiperazin-1-yl, 3,5-Dimethyl-piperazin-1-yl, 6-Amino-3-azabicyclo(3.1.0)hexan-3-yl, 2,8-Diaza-8-bicyclo[4.3.0]nonyl, Octahydro-6H-pyrrolo[3,4-b]pyridin-6-yl, soweit sie den in Anspruch 1 aufgeführten Verbindungen entsprechen.

Beispiele für Reste R2 in den Verbindungen der Formel (II) sind Azetidin-1-yl, 2-Hydroxymethyl-azetidin-1-yl, 2-Aminomethyl-azetidin-1-yl, Pyrrolidin-1-yl, Isoxazolin-1-yl, 2-Methyl-pyrazolidin-1-yl, 3-Hydroxy-pyrrolidin-1-yl, 3-Carboxy-pyrrolidin-1-yl, 3-Amino-pyrrolidin-1-yl, 3-Aminomethyl-pyrrolidin-1-yl, 3-Methylamino-pyrrolidin-1-yl, 3-Ethylamino-pyrrolidin-1-yl, 3-Fluorethylamino-pyrrolidin-1-yl, 3-Trifluorethylaminopynrolidin-1-yl, 3-Methoxyethylamino-pyrrolidin-1-yl, 3-(N-Methyl-N-cyctopropylamino)-pyrrolidin-1-yl, 3-Amino-4-cyclopropyl-pyrrolidin-1-yl, 4-Methyl-3-methylamino-pyrrolidin-1-yl, 3-Cyclopropylamino-4-methyl-pyrrolidin-1-yl, 3-(1-Amino-8-aza-8-bicyclo[4.3.0]nonyl, 3-Aminomethyl-pyrrolidin-1-yl, 3-Aminomethyl-3-trifluormethyl-pyrrolidin-1-yl, 5-Amino-2-aza-2-spiro[4.4]nonyl, 1-Aminomethyt-8-aza-8-bicyclo-[4.3.0]nonyl, 5-Aminomethyl-7-aza-2-oxo-7-bicyclo[3.3.0]octyl, 1-Aminomethyl-7-aza-3-oxo-7-bicyclo[3.3.0]octyl, 2,7-DiaZa-7-bicyclo[3.3.0]octyl, 3,7-Diaza-3-bicyclo[3.3.0]octyl, 2,8-Diaza-8-bicyclo[4.3.0]nonyl, 5,8-Diaza-2-oxo-8-bicyclo[4.3.0]nonyl, 3,8-Diaza-8-bicyclo[4.3.0]nonyl, 2,7-Diaza-7-bicyclo[4.3.0]nonyl, 3,9-Diaza-9-bicyclo[4.3.0]nonyl, 2,7-Diaza-2-bicyclo[3.3.0]octyl, Piperidin-1-yl, 3-Amino-piperidin-1-yl, 3-Amino-4-methyl-piperidin-1-yl, 3,9-Diaza-3-bicyclo[4.3.0]nonyl, 7-Amino-3-aza-3-bicyclo[4.1.0]heptyl, 7-Amino-5-azaspiro[2.4]hept-5-yl oder 7-Methylamino-5-azaspiro[2.4]hept-5-yl.

Beispiele für Verbindungen der Formel (I) oder Formel (II) sind 7-(3-Amino-1-pyrrolidinyl)-8-chlor-1-cyclopropyl-6-fluor-1,4-dihydro-4-oxo-3-chinolincarbonsäure (Clinafloxacin), 1-Cyclopropyl-6-fluor-1,4-dihydro-8-methoxy-7-(3-methyl-1-piperazinyl)-4-oxo-3-chinotincarbonsäure (Gatifloxacin), 7-[(4Z)-3-(Aminomethyl)-4-(methoxyimino)-1-pyrrolidinyl]-1-cyclopropyl-6-fluor-1,4-dihydro-4-oxo-1,8-naphthyridin-3-carbonsäure (Gemifloxacin), 1-Cyclopropyl-6-fluor-1,4-dihydro-5-methyl-7-(3-methyl-1-piperazinyl)-4-oxo-3-chinolincarbonsäure (Grepafloxacin), (3S)-9-Fluor-2,3-dihydro-3-methyl-10-(4-methyl-1-piperazinyl)-7-oxo-7H-pyrido[1,2,3-de]-1,4-benzoxazine-6-carbonsäure (Levofloxacin), 1-Cyclopropyl-6-fluor-1,4-dihydro-8-methoxy-7-[(4aS,7aS)-octahydro-6H-pyrrolo[3,4-b]pyridin-6-yl]-4-oxo-3-chinolincarbonsäure (Moxifloxacin), 5-Amino-1-cyclopropyl-7-[(3R,5S)-3,5-dimethyl-1-piperazinyl]-6,8-difluor-1,4-dihydro-4-oxo-3-chinotincarbonsäure (Sparfloxacin), 7-(3-Amino-1-pyrrolidinyl)-1-(2,4-difluorphenyl)-6-fluor-1,4-dihydro-4-oxo-1,8-naphthyridin-3-carbonsäure (Tosufloxacin) oder 7-(1□,5□,6□- 6-Amino-3-azabicyclo[3.1.0]hex-3-yl)-1-(2,4-difluorphenyl)-6-fluor-1,4-dihydro-4-oxo-1,8-naphthyridin-3-carbonsäure (Trovafloxacin).

Sofern entsprechende physiologisch verträgliche Säureadditionssalze der Verbindungen der allgemeinen Formel (I) und/oder der allgemeinen Formel (II) zum Einsatz kommen, können diese vorzugsweise aus der Gruppe Hydrochlorid, Hydrobromid, Methansulfonat und Toluolsulfonat ausgewählt werden. Sofern entsprechende physiologisch verträgliche Salze der zugrundeliegenden Carbonsäuren zum Einsatz kommen, können diese bevorzugt aus der Gruppe der Alkalisalze, Erdalkalisalze, Ammoniumsalze, Guanidiniumsalze oder Silbersalze ausgewählt werden. Es können auch Gemische aus wenigstens zwei der vorstehend genannten physiologisch verträglichen Salze zum Einsatz kommen.

Die vorstehend genannten Verbindungen der Formel (I) oder Formel (II) sind bekannt und können nach üblichen, dem Fachmann bekannten Verfahren hergestellt werden. Ebenso ist bekannt, dass die Verbindungen der Formel (I) oder Formel (II) antibiotisch wirken und ein antibakterielles Spektrum haben gegen grampositive und gramnegative Keime. Auch ist bekannt, dass die Verbindungen der Formel (I) oder Formel (II) zur systemischen Behandlung von Erkrankungen eingesetzt werden können, die durch gramnegative oder grampositive oder bakterienähnliche Mikroorganismen hervorgerufen werden können. Es ist auch bekannt, dass Ciprofloxacin in topischer Form in der Augenheilkunde angewendet werden kann.

Die Verbindungen der allgemeinen Formel (I), wie sie in Anspruch 1 aufgeführt sind, oder der allgemeinen Formel (II) können zur anspruchsgemäßen topischen und/oder lokalen Behandlung von verschiedenen bakteriell verursachten Erkrankungen und zur Prophylaxe bei Mensch und Tier verwendet werden.

Die Verbindungen der Formel (I), wie sie in Anspruch 1 aufgeführt sind, oder Formel (II) können insbesondere in der Zahnmedizin und/oder zur Verbesserung der Wundheilung in der Allgemeinmedizin oder Tiermedizin verwendet werden. Die erfindungsgemäßen Verwendungen der Verbindungen der Formel (I) gemäß Anspruch 1 oder Formel (II) betreffen bevorzugt a) endodontische Behandlungen wie die topische Behandlung von Pulpitiden auf Grund von kariösen Erkrankungen, die Prophylaxe von Dentinwunden oder die topische Behandlung des infizierten Wurzelkanals und des periapikalen Bereichs, b) die topische Behandlung von Parodontopathien, c) die topische Behandlung von oralen Schleimhaut-Knochenwunden mit gestörter Wundheilung oder die prophylaktische Behandlung, zum Beispiel nach Extraktionen, Zystectomie oder Incisionen beispielsweise auf Grund von Phlegmonen oder Panrlis, d) die topische und/oder lokale Behandlung von postoperativen oder posttraumatischen Wundinfektionen und e) bei der periooperativen Prophylaxe, g) bei Handinfektionen, h) bei der postoperativen Sepsis, i) bei infizierten Ulzera und Gangränen, j) bei akuten und chronischen bakteriellen Hautinfektionen, k) bei sekundär infizierten Dermatosen, l) bei der Akne und Rosazea oder m) bei Schteimhautulzerationen.

Im folgenden werden bevorzugte Verwendungen gemäß der Erfindung beispielhaft auf den Gebieten der Zahnmedizin und der Wundheilung beschrieben.

### Topische Behandlung von Pulpitiden auf Grund von kariösen Erkrankungen.

Die besondere Problematik bei der Behandlung der Pulpa liegt in einer geringen Lymphversorgung, der Stellung als Endorgan mit einem geringen Kollateralkreislauf und dem Einfluß von äußeren Reizen bei Lage in einer starren, unnachgiebigen Höhle. Bei kariösen Defekten dringen die Mikroorganismen Richtung Pulpa vor. Hierbei wird nach Überschreiten der Schmeiz-Dentin-Grenze der Dentinkegel erweicht. Hat der kariöse Prozeß die Hälfte der gesamten Dentinschicht erreicht, ist die Pulpa, auch wenn häufig klinische Symptome noch fehlen, schon histologisch verändert. Ist die Karies weiter fortgeschritten, ohne die Restdentindecke zu durchdringen, sind pulpitische Beschwerden die Regel. Hat die Karies die Pulpa erreicht, sind unterschiedliche histopathologische Formen die Folge. Diese Folgen des Kariesgeschehens sind darauf zurückzuführen, daß Dentin von radiär verlaufenden Kanälchen, den sogenannten Dentinkanälchen durchzogen werden, in denen Fortsätze der Pulpa liegen. Diese Fortsätze der Dentinbildnerzellen (Odontoblasten), die an der Peripherie der Pulpa liegen, dienen unter anderem der Reizleitung. Bekanntlich folgen die Mikroorganismen und deren Toxine den Dentinkanälchen. Hat die Karies die Dentinschicht weitgehend durchdrungen oder die Pulpa erreicht, 10 spricht man von der Caries profunda.

Die besondere Problematik der Diagnostik einer Pulpaerkrankung besteht darin, daß das histologische und das klinische Bild häufig stärker differieren. Da Schmerzempfindungen von den einzelnen Patienten unterschiedlich empfunden und interpretiert werden, besteht immer das Risiko der Fehtdiagnose. Häufig ist 15 die Infektion der Pulpa weiter fortgeschritten als klinisch feststellbar. Bei konventioneller Behandlung besteht dann die Gefahr des Verbleibens von Restkeimen.

Bei der konventionellen und heute üblichen Behandlung der Karies werden zunächst die kariösen Massen entfernt. Danach wird die Kavität beispielsweise mit Wasserstoffperoxid desinfiziert und, je nach Grad der Pulpaentzündung, eine indirekte oder direkte Überkappung vorgenommen. Dies bedeutet, dass eine dünne Restdentindecke stehen gelassen wird oder die eröffnete Pulpa mit einem Medikament überschichtet wird. Hierzu werden entweder Calziumhydroxid oder Zinkoxid-Eugenol eingesetzt. Beide Medikamente sind stark alkalisch, wirken 25 antibakteriell und erzeugen in einem lokalen Bereich der Pulpa eine Ätznekrose. Diese bewirkt, abhängig vom Grad der Vorschädigung und der Resistenzlage, den Anbau von Sekundärdentin. Das Überkappüngsmittel wird mit einem Unterfüllungsmaterial abgedeckt, beispielsweise Zinkphosphatzement. In der gleichen Sitzung oder zu einem späteren Zeitpunkt wird die definitive Füllung 30 zum Beispiel mit einem mit einem Composite-Zement angefertigt. Bei bestimmten Schmerzsymptomen oder bei Pulpen, die nicht mehr auf äußere Reize reagieren, geht man davon aus, daß sich die Pulpa nicht mehr regenerieren kann oder dass sie avital ist. In diesen Fällen wird die Pulpa entfernt und eine Wurzelbehandlung durchgeführt.

Die topische Behandlung von infiziertem Dentin und Pulpitiden mit Antibiotika wird in der wissenschaftlichen Literatur bisher als nicht erfolgreich beschrieben. Als Hauptargument gegen diese Behandlungsmethode wird angeführt, dass die in Frage kommenden Keime (Anaerobier und Aerobier) von den geprüften Antibiotika nur sehr eingeschränkt erfasst wurden. Darüberhinaus wurde festgestellt, daß die früher für diese Indikationen geprüften Antibiotika im allgemeinen nur bakteriostatisch und nicht bakterizid wirken. Hierdurch können resistente Keime überhand nehmen und Überempfindlichkeitsreaktionen gegen ein bestimmtes Antibiotikum ausgelöst werden.

Im Folgenden werden unter Verbindungen der Formel (I) nur die in Anpspruch 1 aufgeführten Verbindungen verstanden.

Es wurde nun überraschenderweise gefunden, dass die Antibiotika der Formel (I) oder Formel (II) bei topischer und/oder lokaler Applikation in den Parodontalspalt in der Zahnheilkunde die bei odontogenen Infektionen vorkommenden Bakterien vollständig bekämpfen und zusätzlich die oben beschriebenen Nachteile der konventionellen Behandlung nicht auftreten. Darüber hinaus wurde gefunden, dass die Verbindungen der Formel (I) oder Formel (II) eine hohe Gewebegängigkeit im odontogenen Bereich haben. Es wurde ferner gefunden, dass die Keime odontogener Infektionen unter der Behandlung mit den Verbindungen der Formel (I) oder Formel (II) keine Neigung zur Ausbildung einer Resistenz aufweisen. Dies ist deshalb von hoher Bedeutung, weil zum Beispiel bei der Caries profunda immer abgewägt werden muss, ob erweichtes Restdentin über der Pulpa belassen werden kann. Auf Grund der vollständigen Eliminierung der auftretenden Keime ergibt sich jetzt eine neue Behandlungsmethode, die darin besteht, dass eine dünne erweichte Dentindecke belassen bleiben kann, wenn die Verbindungen der Formel (I) oder Formel (II) verwendet werden. Mit dieser Behandlungsmethode können infizierte Dentinschichten keimfrei gemacht werden und Infektionen von Pulpen gestoppt werden. Dieses bedeutet, dass die Schmerzreaktionen ausgeschaltet werden und Pulpeninfektionen in bestimmtem Unfang ausheilen. Die Pulpen bleiben vital.

Die topische Anwendung der Verbindungen der Formel (I) oder Formel (II) erfolgte nach der Entfernung des kariösen Dentins. Die Verbindungen der Formel (I) oder Formel (II) wurden beispielsweise in wässriger lösung oder in gelförmiger Konsistenz oder auf einem inerten Träger beispielsweise mittels Wattebausch, appliziert. Die darüber liegende Kavität wurde mit einem Verschlusszement randdicht verschlossen. Der Wattebausch wurde in der Kavität für drei bis sechs Tage belassen. Die Verbindungen der Formel (I) oder Formel (II) können in Konzentrationen von 0,005 mg/mL bis 200 mg/mL eingesetzt werden. Bevorzugte Konzentrationen sind solche im Bereich von 0,5 mg/mL bis 150 mg/mL. Besonders bevorzugte Konzentrationen sind solche im Bereich von 10 bis 100 mg/mL.

Überraschenderweise wurde in einer Vielzahl von Fällen beobachtet, dass die Einwirkung von Verbindungen der Formel (I) oder Formel (II) sogar zu einer Neubildung des Pulpengewebes führte. Dies zeigte sich darin, dass nach Extirpation der infizierten und entzündeten Pulpa die Nervnadel bis zum Foramen physiologicum eingeführt werden konnte. Etwa zwei Wochen nach topischer Applikation einer Verbindung der Formel (I) oder Formel (II) wurde ein Einsprossen von frischem Pulpengewebe beobachtet, dadurch gekennzeichnet, dass das vertikale Kanalvolumen bei instrumenteller Inspektion geringer geworden war und frisches Pulpengewebes wieder kronenwärts in den Kanal eingesprossen war. Dieses Pulpengewebe war vital.

### Prophylaxe von Dentinwunden

Bei der Behandlung von kariösen Defekten oder nach der Präparation von Dentinflächen zur Aufnahme z. B. von Füllungen, Inlays, Onlays, Kronen oder Brücken werden Dentinkanälchen angeschnitten, die in direkter Verbindung zur Pulpa stehen und somit einen Zugang zur Pulpa darstellen. Jede präparierte Dentinfläche ist von einer organischen Schicht und Präparationsrückständen (smear layer) bedeckt. Wenn die Präparationsarbeit auch im kariös veränderten Dentin erfolgt, enthält diese in der Regel Mikroorganismen. Es ist gebräuchlich, die Dentinwunde mit Wasserstoffperoxid auszuwaschen und anschließend vorsichtig mit dem Luftbläser zu trocknen. Auch wenn die Kavitätenwände nach Entfernen der Karies und Präparation der Kavität "sondenhart" sind und die Kavität mit desinfizierenden Mitteln behandelt worden ist, kann nicht ausgeschlossen werden, dass Keime in Dentinkanälchen schon weiter in Richtung Pulpa vorgedrungen und für desinfizierende Mittel nicht zu erreichen sind. Hierdurch können Dentinirberempfndlichkeiten und später Sekundärkaries verursacht werden.

Es wurde nun gefunden, dass die prophylaktische topische Verwendung der Verbindungen der Formel (I) oder Formel (II) nach jeder Präparation bei kariösen Defekten im Dentinbereich auch weit in Dentinkanälchen vorgedrungene Keime abtöten können und sie damit einen vorteilhaften Effekt auf die zahnerhaltenden Maßnahmen aufweisen. Dazu werden nach der Entfernung der kariösen Bereiche die Verbindungen der Formel (I) oder Formel (II) auf die präparierten Kavitäten aufgetragen und eingerieben. Dies geschieht in Form von Lösungen, Gelen oder Suspensionen, die die Ausbildung eines lokalen antibiotisch wirksamen Depots vor der Legung der Unterfüllung auf und/oder im Fundament der Inlays, Onlays, Kronen oder Brücken bewirken. Dies führt nicht nur zu einem effektiven Abtöten der restlichen Erreger auf der Oberfläche der Kavität, sondern auch in den Dentinkanälchen. Von Vorteil ist weiterhin, dass hierbei nicht, wie dies bei heutigen Maßnahmen mit Dentinschutz- und Imprägniermitteln häufig der Fall ist, die Tubulistruktur verändert oder denaturiert wird. Die derart erreichten keimfreien Bedingungen bewirken einen dauerhaften Erfolg prothetischer Maßnahmen, da Dentinüberempfindlichkeiten und später Sekundärkaries wirksam vermieden werden.

Die Applikation der Verbindungen der Formel (I) oder Formel (II) kann anstelle der desinfizierenden Behandlung mit Wasserstoffperoxid erfolgen oder aber ergänzend zu dieser erfolgen. Die Verbindungen der Formel (I) oder Formel (II) können in gelöster Form oder als Gel appliziert werden, gegebenenfalls in Gegenwart von Lösungsvermittlern, die ein tiefes Eindringen der Chemotherapeutika in die Dentinkanälchen fördern. Gele werden bei provisorischer Versorgung von Dentinwunden und Lösungen bevorzugt vor definitiver Befestigung zum Beispiel von Kronen und Brücken angewendet. Die Lösungen oder Gele der applizierten Chemotherapeutika werden auf die präparierten Dentinoberflächen aufgetragen und leicht eingerieben. Danach werden Reste entfernt, beispielsweise mit einem Wattebausch oder Luftstrom. Anschließend können Inlays, Onlays, Kronen oder Brücken fixiert werden. Die Verbindungen der Formel (I) oder Formel (II) können in Konzentrationen von 0,005 mg/mL bis 200 mg/mL eingesetzt werden. Bevorzugte Konzentrationen sind solche im Bereich von 0,5 mg/mL bis 150 mg/mL. Besonders bevorzugte Konzentrationen sind solche im Bereich von 10 bis 100 mg/mL.

### Topische Behandlung des infizierten Wurzelkanals und des periapikalen Bereichs.

Bei der konventionellen und heute üblichen Methode der topischen Behandlung des infizierten Wurzelkanals und des periapikalen Bereichs sind Misserfolge dann zu erwarten, wenn nach umfangreichen Infektionen der Pulpa und damit des Wurzelkanales die äußerst schwierige Diagnosestellung falsch war. In diesen Fällen befinden sich pathogene Keime in den Seitenabzweigungen der Pulpa, im apikalen Delta, im periapikalen Bereich und in der äußeren Dentinschicht der Pulpahöhle. Bekanntlich ist es in vielen dieser Kanalregionen auf Grund der anatomischen Gegebenheiten instrumentell nicht möglich, infiziertes Gewebe vollständig zu entfernen. In der Regel gelingt es nicht, instrumentell die vielfältigen Verzweigungen, beispielsweise ein apikales Delta oder seitliche Verzweigungen, die Markkanäle, zu erreichen und aufzubereiten. Die gebräuchlichen Desinfektionsmittel sind häufig ebenfalls erfolglos. Eine besonders schwierige Situation liegt dann vor, wenn die Keime über das Wurzelkanalloch (foramen apikale) hinaus vorgedrungen sind. Dieser periapikale Bereich ist mit Wurzelkanalinstrumenten oder Desinfektionsmitteln in Form von Wurzelkanaleinlagen nicht zu erreichen.

Die konventionelle Behandlung besteht darin, dass die Pulpa mit einer Nervnadel entfernt wird und mit Hilfe von schneidenden Wurzelkanalinstrumenten der Kanal aufbereitet wird. Hierunter ist ein Erweitern des Kanallumens und eine Abtragung der infizierten Kanalwandung zu verstehen. Kombiniert wird die mechanische Aufbereitung mit dem Einsatz chemischer Mittel. Nach Abschluß dieser Behandlung erfolgt die definitive Wurzelfüllung.

Es wurde nun überraschenderweise gefunden, dass die Verbindungen der Formel (I) oder Formel (II) auch zur topischen Behandlung des infizierten Wurzelkanales und des periapikalen Bereichs verwendet werden können. Dabei wurde gefunden, dass die Verbindungen der Formel (I) oder Formel (II) alle im Wurzeikanal und im periapikalen Bereich vorkommenden Keime abtöten, auch in allen mechanisch nicht zugänglichen Verzweigungen des Wurzelkanals und über die Wurzelspitze hinaus im periapikalen Bereich. Es wurden keine bakteriellen Resistenzen bei Verwendung der Verbindungen der Formel (I) oder Formel (II) gefunden. Dieses Verhalten der Verbindungen der Formel (I) oder Formel (II) ermöglicht es, auf eine extensive Aufbereitung in der bisher praktizierten Form verzichten zu können.

Bei der klinischen Anwendung der Verbindungen der Formel (I) oder Formel (II) werden zunächst die Pulpenreste entfernt. Die Kanalaufbereitung erfolgt, soweit möglich, schonend. Anschließend werden die Kanäle mit Wasserstoffperoxid gespült. Auf den Einsatz anderer Desinfektionsmethoden kann verzichtet werden. Danach werden die Verbindungen der Formel (I) oder Formel (II) in flüssiger Darreichungsform mittels Kanüle unter Druck in den Wurzelkanal appliziert.

Anschließend werden die Verbindungen der Formel (I) oder (II) in gelöster Form oder in gelförmiger Darreichungsform nachgespritzt. Der Wurzelkanal wird dann im Kronenbereich zunächst mit einem Wattebausch und anschließend mit Zinkphosphat-Zement randdicht verschlossen. Die Antibiotika verbleiben bei nicht gangränösen Kanälen für 3 Tage in den Kanälen. Bei gangränösen Kanälen hat es sich als günstig erwiesen, wenn eine erneute Applikation für weitere drei Tage erfolgt. Diese Behandlung führt zu einer erfolgreichen Therapie infizierter Wurzelkanäle und des apikalen Bereichs. Die Verbindungen der Formel (I) oder Formel (II) können in Konzentrationen von 0,005 mg/mL bis 200 mg/mL eingesetzt werden. Bevorzugte Konzentrationen sind solche im Bereich von 0,5 mg/mL bis 150 mg/mL. Besonders bevorzugte Konzentrationen sind solche im Bereich von 10 bis 100 mg/mL.

Die topische Applikation in den Wurzelkanal kann vorteilhaft auch durch die lokale Injektion einer Lösung der Verbindungen der Formel (I) oder Formel (II) in den Parodontalspalt ergänzt werden. Bei dieser Applikationstechnik, die von der intraligamentalen Anästhesie bekannt ist, wird der Zahn im gesamten Wurzelbereich komplett von der antibakteriellen Lösung der Verbindungen der Formel (I) oder Formel (II) umflutet. Dies führt zu einem effektiven Abtöten der Keime, die in den Parodontalspalt und in den periapikalen Bereich eingedrungenen Keime. Bevorzugte Applikationsformen der intraligamentalen Injektion sind Lösungen der Verbindungen der Formel (I) oder Formel (II) in Konzentrationen von 0,005 mg/mL bis 200 mg/mL. Bevorzugte Konzentrationen sind solche im Bereich von 0,5 mg/mL bis 150 mg/mL. Besonders bevorzugte Konzentrationen sind solche im Bereich von 1 bis 100 mg/mL.

Ein besonderer Vorteil der Verwendung der Verbindungen der Formel (I) oder Formel (II) besteht darin, dass es möglich ist, Zähne zu erhalten, die bisher häufig extrahiert werden mussten oder bei denen eine Wurzelspitzenresektion vorgenommen werden mußte. Es ist zu erwarten, dass sich die Folgekosten, z.B. durch Anfertigung von Zahnersatz, deutlich senken lassen.

### Topische Behandlung von Parodontopathien.

Parodontopathien sind häufig die Folge mangelhafter Mundhygiene und heilen in vielen Fällen nach lokalen Maßnahmen, wie z.B. Belagsentfernung aus. Der Erfolg ist jedoch abhängig von der Taschentiefe und davon, ob es gelingt, durch supragingivales Scaling die pathogenen Keime zu entfernen. Ist diese Behandlungsmaßnahme nicht erfolgreich, wird die Kombination mit der lokalen Applikation von Antibiotika empfohlen. Gebräuchlich ist die lokale Anwendung von Tetracyclinen mit Hilfe von Fäden. Diese werden für mehrere Tage in die Zahnfleischtasche geschoben. Der Vorteil dieser Methode liegt darin, daß in vielen Fällen ein operativer Eingriff umgangen werden kann.

Überraschenderweise wurde nun gefunden, dass auch die Verbindungen der Formel (I) oder Formel (II) zur Behandlung von Parodontopathien geeignet sind. Dazu werden Fäden oder sogenannte Chips, die mit Lösungen oder gelförmigen Zubereitungen der Verbindungen der Formel (I) oder Formel (II) getränkt worden sind, in die Zahnfleischtasche eingelegt. Alternativ können auch Medikamententräger in Form von Trays, die Zähne und Zahnfleisch abdecken, angewendet werden. Vor deren Anwendung erfolgt eine Instillation des gelförmigen Applikationen der Verbindungen der Formel (I) oder Formel (II) in die Zahnfleischtaschen. Zusätzlich kann das Gel, das die Verbindungen der Formel (I) oder Formel (II) enthält, auch in den Medikamententräger gegeben werden. Diese Verwendung der Medikamententräger ist deshalb von Vorteil, weil die Behandlungsdauer aufgrund des schnellen Wirkeintritts auf etwa 15 bis 30 Minuten beschränkt werden und deshalb direkt in der Praxis erfolgen kann. Die Verbindungen der Formel (I) oder Formel (II) können in Konzentrationen von 0,005 mg/mL bis 200 mg/mL eingesetzt werden. Bevorzugte Konzentrationen sind solche im Bereich von 0,5 mg/mL bis 200 mg/mL. Besonders bevorzugte Konzentrationen sind solche im Bereich von 10 bis 150 mg/mL.

Es ist auch möglich, zur Behandlung von Parodontopathien die Verbindungen der Formel (I) oder Formel (II) durch intraligamentale Injektion zu verabreichen, wobei Konzentrationen im Bereich von 0,005 mg/mL bis 200 mg/mL eingesetzt werden können, bevorzugt von 0,5 mg/mL bis 200 mg/mL. Eine intraligamentale Injektion ist bei den heute gebräuchlichen Antibiotika wie den Tetracyclinen nicht sinnvoll, da diese Verbindungen mit vorwiegend bakteriostatischer Wirkung wegen des schnellen Flüssigkeitsaustausches im Parodontalspalt schnell ausgewaschen werden und ihre Wirkung nicht entfalten können. Tetracycline können deshalb nur über Depotformulierungen ihre Wirkungen entfalten. Im Gegensatz dazu wirken die Verbindungen der Formel (I) oder Formel (II) sehr schnell und dazu noch bakterizid, so dass ihre Vennreilzeit im Parodontalspalt zur Abtötung der Keime ausreicht.

Die Behandlungsverläufe waren dadurch gekennzeichnet, dass sie sehr effektiv war, indem das gesamte bei Parodontopathien beteiligte bakterielle Keimspektrum abgedeckt wurde. Die Heilungsraten waren sehr hoch. Die Behandlung verlief in der Regel auch deutlich schneller als bei der bisher üblichen. Die klinischen Erfolge traten in der Regel schon nach dreimaliger Liegezeit ein. Die Erfolgsaussichten dieser neuen Methode zur Behandlung von Parodontopathien sind durch Verwendung von Verbindungen der Formel (I) oder Formel (II) wesentlich verbessert worden. Durch dieses Vorgehen kann in vielen Fällen ein operativer Eingriff umgangen werden.

Überraschenderweise wurde bei der Applikation der Verbindungen der Formel (I) und (II) in der Zahnfleischtasche gefunden, dass das Gewebe des Zahnfleisches sehr schnell regenerierte, eine straffe Konsistenz erreichte und nicht mehr blutete. Die topische und/oder lokale Applikation der Verbindungen der Formel (I) und (II) führt demnach zu einer schnellen Regenerierung der Gewebe des Zahnhalteapparates. Dies führt zu einem schnellen Nachwachsen der zahnstützenden Gewebe und einem Reattachement der Zähne.

### Topische Behandlung von Schleimhaut-Knochenwunden.

Bakterielle Infektionen des Knochens und der Weichteile im Mund- und Kieferbereich und im Gesicht haben häufig odontogene Ursachen. Ausgangspunkt sind meistens pulpentote Zähne, Wurzelreste, dentogene Zysten, Dentitio difficilis und progressive Parodontopathien. Dentogene Abzesse werden üblicherweise chirurgisch eröffnet und bis zur Ursachenbeseitigung drainiert. Die verschiedenen Infektionen im Mundbereich erfordern unterschiedliche Therapiemaßnahmen. So kann meistens, z.B. bei alveolarfortsatznahen Infektionen, auf eine systemische Chemotherapie verzichtet werden, während z.B. die Behandlung von Phlegmonen immer die systemische Gabe von Antibiotika erfordert. Die Kombination mit der topischen Anwendung von Antibiotika ist jedoch in der Regel angezeigt.

Es wurde gefunden, dass die Verbindungen der Formel (I) oder Formel (II) auch zur topischen Behandlung von Schleimhaut- und Knochenwunden verwendet werden können. Sie haben einen förderlichen Einfluss auf die Therapie. Durch den topischen Einsatz der Verbindungen der Formel (I) oder Formel (II) kommt es in der Regel zu einem raschen Abklingen der Entzündungssymptome und einem schnellen Einsetzen der Heilung. Es wurde beobachtet, dass der Heilungsprozess in der Regel deutlich schneller ablaufen als in den heute gebräuchlichen Methoden. Die Verbindungen der Formel (I) oder Formel (II) haben also eine ausgesprochen positive, das heißt beschleunigende Wirkung auf den Wundheilungsprozess.

Die Applikation erfolgt durch Spülungen und/oder Streifeneinlagen, die mit Verbindungen der Formel (I) oder Formel (II) getränkt sind. Sie sind beispielsweise anzuwenden bei postoperativen Infektionen nach zahnärztlichchirurgischen Eingriffen (Extraktionen). Hierzu wird das die Verbindung der Formel (I) oder Formel (II) enthaltende Gel mittels Spritze direkt in die Extraktionswunde oder auf einen Kollagenschwamm, der in der Extraktionswunde verbleibt, gegeben. Die Wunde wird für etwa eine halbe Stunde durch Beißen auf einen Tampon verschlossen. Die prophylaktischen Applikationen sind besonders nach Extraktionen von beherdeten Zähnen erfolgreich gewesen.

Zum Durchspülen von Fistelgängen sind flüssige Darreichungsformen geeignet, die die Verbindungen der Formel (I) oder Formel (II) in Konzentrationen von 0.005 mg/mL bis 250 mg/mL enthalten. Bevorzugte Konzentrationen sind solche von 0,5 bis 100 mg/mL. Zum Belegen von Streifen sind Gele, die die Verbindungen der Formel (I) oder Formel (II) enthalten, geeignet, aber auch wässrige Lösungen oder Suspensionen. Diese Applikationen können die Verbindungen der Formel (I) oder Formel (II) in Konzentrationen von 0,005 mg/mL bis 200 mg/mL enthalten. Bevorzugte Konzentrationsbereiche für Lösungen sind solche von 0,1 bis 50 mg/mL Bei Streifeneinlagen sind gelförmige Darreichungsformen in den Konzentration von 25 - 150 mg/mL bevorzugt.

### Wundversorgung

Postoperative Wundinfektionen können allgemein bei unzureichender Infektionsprophylaxe nach chirurgischen Eingriffen auftreten. Posttraumatische Wundinfektionen können durch Schnitte, Stiche, Quetschungen, Bisse oder Schüsse hervorgerufen werden. Eine lokale, perioperative Prophylaxe kann bei aseptischen Operationen mit leichtem Infektionsrisiko erfolgen. Zusätzlich zur systemischen perioperativen Prophylaxe kann auch eine lokale perioperative Prophylaxe angewandt werden, zum Beispiel bei Infektionen mit erhöhtem Infektionsrisiko wie Implantationen, bei Herzoperationen, bei Transplantationen, bei neurochirurgischen Operationen, bei Operationen in stark kontaminiertem Gebiet wie Mundhöhle, Ösophagus, Rektum oder Kolon, bei Hysterektomie, bei Gallenwegsoperationen, bei Operationen von Patienten mit Abwehrschwäche oder bei Amputationen. Handinfektionen sind beispielsweise Panaritium cutaneum, Panaritium subcutaneum, Panaritium ossale, Panaritium articulare oder Tendovaginitis purulenta. Bei der postoperativen Sepsis können die infizierten Wunden durch topische Gabe der antibakteriellen Mittel weitgehend oder vollständig keimfrei gemacht werden. Gangräne können zusätzlich zur Therapie mit parenteralen Antibiotika durch lokale Behandlung mit Antibiotika behandelt werden. Akute bakterielle Hautinfektionen sind beispielsweise Pyodermien, Erysipel, Furunkel, Karbunkel, Phlegmonen, Abszesse, Ulcus cruris, der diabetische Fuß, infizierte Dekubitalulzera, hämorrhagische Blasen, Erysipeloide, oder Erythrasma. Beispiele chronischer bakterieller Hautinfektionen sind Lupus vulgaris, Schwimmbad-Granulome, Buruli-Ulkus oder Aktinomykose. Bakterielle Sekundärinfektionen treten beispielsweise bei Virusinfektionen wie Herpes simplex, Herpes zoster oder Varizellen auf. Bakterielle Sekundärinfektionen von Dermatosen treten beispielsweise bei Ekzemen, Neurodermitis im Exsudationsstadium, blasenbildenden Dermatosen oder Kontaktdermatitis auf. Leichtere und mittlere Formen der Akne und Rosazea können auch lokal behahdlet werden. In allen genannten Fällen kann die lokale Gabe der Verbindungen der Formel (I) oder Formel (II), entweder allein oder aber additiv zu einer systemischen Applikation erfolgen.

Die lokale Gabe der Verbindungen der Formel (I) oder Formel (II) hat sich als vorteilhaft erwiesen, da wegen der breiten Wirksamkeit dieser Verbindungen vorteilhaft auch Mischinfektionen behandelt werden können. Bisherige topisch applizierte Antibiotika haben nur ein eingeschränktes Wirkspektrum und sind damit weniger wirksam. Weitere Vorteile der Verbindungen der Formel (I) oder Formel (II) sind der sehr schnelle Eintritt der antibakteriellen Wirkung. Dies ermöglicht einen therapeutischen Erfolg schon während des Aufenthaltes des Patienten in der Praxis. Diese Verbindungen der Formel (I) oder Formel (II) haben eine ausgeprägte bakterizide Wirkung und sind damit den heutigen, topisch applizierten Antibiotika überlegen, die oft nur eine bakteriostatische Wirkung haben und demnach wesentlich häufiger und länger angewendet werden müssen. Weitere Vorteile der Verbindungen der Formel (I) oder Formel (II) gegenüber heutigen Lokalantibiotika ist ihre gute Gewebegängigkeit. Ihre Penetration durch die intakte Haut ermöglicht auch die erfolgreiche örtliche Lokalbehandlung von tiefer liegenden Hautinfektionen. Ebenfalls als Vorteil der Verbindungen der Formel (I) oder Formel (II) ist anzusehen. dass sie ein wesentlich geringeres Potential zur bakteriellen Resistenzbildung aufweisen als herkömmliche Lokalantibiotika. Dadurch lassen sie sich wesentlich sicherer anwenden. Ein weiterer Vorteil der Verbindungen der Formel (I) oder Formel (II) ist eine allgemein zu beobachtende beschleunigende Wirkung auf die Wundheilung. Zusätzliche Vorteile der lokalen Verwendung von Verbindungen der Formel (I) oder Formel (II) sind die Verhinderung von Komplikationen wie Lymphangitis, Sepsis oder chronische Lokalinfektionen.

Die besondere Wirksamkeit der Verbindungen der Formel (I) oder Formel (II) bei topischer Applikation hat sich überraschenderweise auch bei der Therapie des diabetischen Fuß-Syndroms gezeigt. Nach dem heutigen Stand der Medizin gilt, dass jede Läsion mit entzündlichem Lokalbefund mit und ohne systemische Infektionszeichen einer sofortigen und breitabdeckenden Antibiose bedarf. Dabei ist zu berücksichtigen, daß bei dem entzündlichen Geschehen regelhaft eine Mischinfektion von grampositiven und gramnegativen Keimen sowie von Anaerobiem und Aerobiem vorliegt. Initial hat sich die systemische Gabe von Amoxicillin, Ctavutansäure oder Clindamycin jeweils in Kombination mit einem Gyrasehemmer bewährt. Entsprechend des Ergebnisses des vor Antibiotikagabe abgenommenen Wundabstriches kann dann die Antibiose gezielt durchgeführt werden. Kontrovers wird jedoch die notwendige Dauer der Antibiotikatherapie, insbesondere bei osteomyelitischen Defekten, diskutiert Eine monatelange systemisch hochdosierte Antibiotikagabe erscheint sinnlos, wenn radiologisch eine nachweisbare Abheilungstendenz der Osteolysen nicht zu erkennen ist; hier wird eine chirurgische Intervention unumgänglich sein.

Bei der heute üblichen Therapie des diabetischen Fußes gilt, dass eine essentielle Voraussetzung für eine komplikationslose Abheilung eine infektionsfreie Wunde ist. Daher ist beim Vorliegen einer infizierten Wunde die schnelle und zuverlässige Infektionsbehandlung oberstes Gebot. Eine lokale oder systemische Antibiotikatherapie birgt das Risiko der Allergie und der Resistenzentwicklung. Besonders bewährt haben sich Verbände, bestehend aus Aktivkohle und elementarem Silber. Das nichttoxische elementare Silber erlaubt eine sehr starke lokale Infektionsbekämpfung. Die Aktivkohle bindet Mikroorganismen und Zelldetritus und erlaubt das Entfernen der ungewünschten Partikel beim Verbandwechsel. Resistenzentwicklungen, lokale Reizungen oder Allergien sind dabei ausgeschlossen, gleichzeitig wird das notwendige feuchte Milieu gewährleistet. Als Desinfektionsmittel haben Farbstoffe bei der modernen Wundbehandlung, mit Ausnahme von PVP-Jod-Komplexen, keine Bedeutung mehr. Kaliumpermanganat ist problematisch in der Dosierung und kann zu schweren Hautverätzungen führen. Ethacridinlaktat weist eine hohe Allergierate und nur eine beschränkte antimikrobielle Wirksamkeit auf. Das quecksilberhaltige Merbromin ist hochtoxisch, beeinträchtigt die Granulation und ist problematisch bei der Entsorgung. Andere Farbstoffe, wie z.B. Brillantgrün, Methylviolett und Fuchsin, sind wegen ihrer geringen Wirksamkeit, vor allem aber wegen des epithelschädigenden Effektes obsolet.

Demnach besteht nach dem heutigen Stand der Technik auch bei der Behandlung des diabetischen Fußes ein Bedürfnis nach topisch und/oder lokal anwendbaren antibiotisch wirksamen Mitteln, die eine hohe Aktivität gegen die in Wunden auftretenden Keime aufweisen, die schnell wirksam sind, die lokal gut verträglich sind, die eine geringe Neigung zur Erzeugung antibiotischer Resistenz hervorrufen, die bei topischer und/oder lokaler Anwendung einfach zu verabreichen sind, die bei topischer/lokaler Gabe eine minimale systemische Belastung des Organismus bewirken, die eine gute Gewebegängigkeit aufweisen, die auch zur Behandlung von tiefen Infektionen geeignet sind und die darüber hinaus den Prozess der Wundheilung beschleunigen.

Es wurde nun gefunden, dass auch bei der topischen Applikation der Verbindungen der Formel (I) oder Formel (II) ein Erfolg bei der Therapie des diabetischen Fuß-Syndroms erzielt werden konnte. Dies zeigte sich besonders darin, dass bei Patienten mit schweren Mikroangiopathien der Füße mit diabetischen Fußsyndromen die systemischen Behandlungsversuche mit Avalox 400 (Moxifloxacin) und/oder Clont 400 (2-Methyl-5-nitro-1H-imidazole-1-ethanol) ohne nennenswerte Befundänderungen vertiefen, da diese Wirkstoffe wegen ausgeprägter Mikroangiopathien nach systemischer Gabe nur sehr geringe Gewebekonzentrationen erreichen konnten. Überraschenderweise führte die topische Applikation der Verbindungen der Formel (I) oder Formel (II) jedoch zu signifikanten Verbesserungen des Krankheitsgeschehens. Es konnten Ulcera unterschiedlicher Schweregrade (Ulcera D I bis D V) behandelt werden.

Schmierige Ulcera wurden sauber, verkrusteten, ihre Größe verringerte sich und sie heilten sogar vollkommen ab. Diese Behandlungserfolge wurde erzielt nach mehrmaliger topischer Applikation der Verbindungen der Formel (I) oder Formel (II) in wöchentlichem Abstand. Dabei konnten die Verbindungen der Formel (I) oder Formel (II) direkt in gelöster Form oder beispielsweise als Gel oder mittels getränkter Kompressen oder Verbände auf die Wunde aufgetragen werden. Diese Verbindungen der Formel (I) oder Formel (II) können in Konzentrationen von 0,005 mg/mL bis 200 mg/mL auf die Wunde aufgetragen werden, bevorzugte Konzentrationsbereiche für Lösungen oder Gele sind solche von 0,1 bis 150 mg/mL. Bei getränkten Kompressen oder Verbänden können gelförmige Darreichungsformen in den Konzentration von 25 - 150 mg/mL verwendet werden.

Es wurde ferner gefunden, dass die lokale und/oder topische Anwendungen der Verbindungen der Formel (I) oder Formel (II) auch in Tiermedizin von Nutzen ist. Neueren Untersuchungen zufolge benötigen in vielen Ländern zwei Drittel der Hunde und mehr als 80 % der Katzen über vier Jahre einer Zahnbehandlung. Mehr als 10 % der Katzen und 7,5 % der Hunde über 4 Jahre leiden darüber hinaus an schweren Parodontitiden, die zu Nieren-, Leber- und Herzinfektionen führen können. Überraschenderweise wurde nun gefunden, dass auch die Verbindungen der Formel (I) oder Formel (11) zur Behandlung von Zahnerkrankungen von Tieren geeignet sind. Bei den endodontischen Behandlungen können die gleichen Verfahren und Wirkstoffkonzentrationen der Verbindungen der Formel (I) oder Formel (II) angewendet werden, wie sie weiter oben für die Behandlungen in der humanen Zahnmedizin erarbeitet worden sind. Zur Behandlung von Parodontopathien werden den Tieren Fäden, die mit Lösungen oder gelförmigen Zubereitungen der Verbindungen der Formel (I) oder Formel (II) getränkt worden sind, in die Zahnfleischtasche eingelegt. Alternativ können auch Silicon-Trays angewendet werden. Die Verbindungen der Formel (I) oder Formel (II) können in Konzentrationen von 0,005 mg/mL bis 200 mg/mL eingesetzt werden. Bevorzugte Konzentrationen sind solche im Bereich von 0,5 mg/mL bis 200 mg/mL. Besonders bevorzugte Konzentrationen sind solche im Bereich von 10 bis 150 mg/mL.

Da bei Tieren die Applikation von Fremdkörpern im Mundbereich mit längerer Liegedauer zu Abwehrreaktionen führen kann, ist die Behandlung von Parodontopathien mit Verbindungen der Formel (I) oder Formel (II) durch direkte Instillation in die Zahnfleischtasche, durch intraligamentale Injektion oder mittels eines Medikamententrägers, beispielsweise eines Silicon-Trays, häufig von Vorteil. Im letzteren Fall wird den zu behandelnden Tieren unter Narkose ein Kloß aus knetbarer Silicon-Abdruckmasse, wie er in der humanen Zahnmedizin gebräuchlich ist, gegen den Oberkiefer gedrückt. Nach Schließen des Mauls werden die Unterkieferzähne abgeformt und durch Andrücken des noch plastischen Materials die gesamten Gingivalsaumbereiche vestibulär und oral überlappend abgedeckt. Nach Erhärtung wird der Gesamtabdruck der Ober- und Unterkieferzähne und der Zahnfleischbezirke entnommen und eine Verbindung der Formel (I) oder Formel (II) in die Zahnfleischtaschen instilliert und gegebenenfalls in die Kavitäten des Abdrucks gegeben. Das Abformstück wird wieder auf die Kiefer der Tiere gesetzt. Das Maul wird für etwa 15 bis 30 Minuten geschlossen gehalten, wobei die Verbindungen der Formel (I) oder Formel (II) topisch mit dem Gewebe in Kontakt kommen.

Die topische und/oder lokale Applikation der Verbindungen der Formel (I) und (II) führt zu einem schnellen Abtöten der Bakterien und zu einer schnellen Regenerierung der Gewebe des Zahnhalteapparates. Dies führt zu einem schnellen Nachwachsen der zahnstützenden Gewebe und einem Reattachement der Zähne. Ein weiterer Vorteil dieser Behandlungsmethode ist, dass der intensive Mundgeruch der Tiere wirksam und kausal bekämpft wird. Die Verbindungen der Formel (I) oder Formel (II) können als Lösungen oder als Gele in Konzentrationsbereichen von 0,005 mg/mL bis 200 mg/mL eingesetzt werden können, bevorzugt von 0,5 mg/mL bis 200 mg/mL.

Die Verbindungen der Formel (I) oder Formel (11) können auch in Kombinationen mit anderen Antiinfektiva wie antibakteriellen, antifungalen oder antiviralen Wirkstoffen eingesetzt werden.

Die Verbindungen der Formel (I) oder Formel (II) können in Konzentrationen von 0,005 mg/mL bis 200 mg/mL eingesetzt werden. Bevorzugte Konzentrationen sind solche im Bereich von 0,5 mg/mL bis 200 mg/mL. Besonders bevorzugte Konzentrationen sind solche im Bereich von 10 bis 150 mg/mL.

Die Verbindungen der Formel (I) oder Formel (II) können als Lösungen, Gele, Suspensionen, Emulsionen, Liposomen oder in Mizellen eingesetzt werden. Lösungen sind beispielsweise Lösungen in Wasser oder wässrige Lösungen in Gegenwart von Löslichkeitsverbesserern. Löslichkeitsverbesserer sind beispielsweise Salze, Polyole, Zuckeralkohole, Polyglycole oder Kosolventien wie Glycerin, Ethylenglycol, Propylenglycol, Furfural, N,N-Dimethylfomamid, Methanol, Ethanol, i-Propanol, n-Propanol oder Aceton. Wässrige Gele werden durch Zusatz von Gelbildnern wie beispielsweise Pektinen, Ethylenglycolmonomethacrylatgel, Alginaten, Hydroxyethylcellulose, Carboxymethylhydroxyethylcellulose, Polyglycerylmethacrylate oder Polysacchariden hergestellt. Geeignete Zusätze sind auch Verdicker wie Cellulose, Alkylcellulose, Hydroxyethylcellulose, Agar-Agar, Carboxymethyl-Guar, Celluloseether oder hydrotrope Lösungsvermittler wie Ethylendiamin, Harnstoff oder Cyclodextrine. Die galenischen Formen können auch Solubilisatoren wie Tenside oder Konservierungsmittel enthalten. Bestandteile von Suspensionen können beispielsweise Traganth, Cellulose, Netzmittel, Glycole, Polyole, Schleimstoffe oder Celluloseether sein. Bestandteile von Emulsionen können Emulgatoren wie Polysorbate, Tenside, Lecithine, Schleimstoffe, Gelatine oder Carboxymethylcellulose sein. Geeignete Formen der Verabreichung können auch Inserte zum Einlegen in die Zahntaschen sein, die aus einem inerten Trägermaterial bestehen und die mit dem Wirkstoff und gegebenenfalls weiteren Hilfsstoffen imprägniert sind und den Wirkstoff unter Herauslösen allmählich freigeben. Formen der Verabreichung für Wurzelfüllungen können beispielsweise Tampons, Wattebausche oder Schaumstoffpellets sein. Die Applikation bei Weichteilinfektionen kann mit Streifen oder Fadeneinlagen erfolgen. Es können auch Arzneistoffe oder Hilfsstoffe zur osmotischen Angleichung verwendet werden. Weitere Hilfsstoffe zur Formulierung der Verbindungen der Formel (I) oder Formel (II) können Antioxidantien, Chelatbildner, Desinfektionsmittel, Dispergiermittel, Emulsionsstabilisatoren, Hydrokolloide, Konservierungsmittel, Lösungsvermittler, Netzmittel, quartäre Ammoniumverbindungen, Stabilisatoren, Suspendiermittel oder Verdickungsmittel sein. Die vorstehend genannten Bestandteile können auch in Kombinationen untereinander verwendet werden.

Geeignete stabile gelartige Formulierungen sind solche, die neben den Verbindungen der Formel (I) oder Formel (II) aus Polyethern und modifizierten Cellulosen und Wasser aufgebaut sind. Bevorzugte Formulierungen für Gele sind solche, die die Verbindungen der Formel (I) oder Formel (II) in Gemischen aus Propylenglycol, Tween-20-Lösung und Muc. Hydroxyethylcell. enthalten. Bevorzugte Zusammensetzungen bestehen aus Verbindungen der Formel (I) oder Formel (II) in Mengen von 0,001 bis 100 mg/mL, Polypropylenglycol in Mengen von 5 bis 250 mg/mL, Tween-20-Lösung 1% in Mengen von 5 bis 200 mg/mL und Muc. Hydroxyethyiceil. ad 1g/mL enthalten. Besonders bevorzugte gelartige Formulierungen bestehen aus den Verbindungen der Formel (I) oder Formel (II) mit 1 bis 100 mg/mL. Propylenglycol mit 50 bis 200 mg/mL, Tween-20-Lösung 1% mit 3 bis 150 mg/mL und Muc. Hydroxycell ad 1 g/mL.

Die folgenden Beispiele dienen der Erläuterung der vorliegenden Erfindung, schränken aber den allgemeinen Erfindungsgedanken nicht ein.

### BEISPIELE

### Beispiele verschiedener Formulierungen

### Beispiel 1:

| Moxifloxacin-HCl-Lösung | |
|---|---|
| Moxifloxacin-Hydrochlorid | 500 mg |
| aqua pro injectione | ad 100 ml |

### Beispiel 2:

| hochviskose Formulierung: Moxifloxacin-HCl-Gel | |
|---|---|
| Moxifloxacin-Hydrochlorid | 1,0 g |
| Hydroxyethylcellulose | 0,5 g |
| Propylenglycol | 1,5 g |
| dest. Wasser zu | 10,0 g |

### Beispiel 3:

| niedrigviskose Formulierung mit Stabilisator: Moxifloxacin-HCl-Gel | |
|---|---|
| Moxifloxacin-Hydrochlorid | 1,0 g |
| Hydroxyethylcellulose | 0,25 g |
| Propylenglycol | 1,5 g |
| Tween-20-Lösung 1 %-ig | 1,0 g |
| dest. Wasser zu | 10,0 g |

### Beispiel 4:

| Moxifloxacin-HCl-Gel | |
|---|---|
| Moxifloxacin-Hydrochlorid | 0,1 g |
| Hydroxyethylcellulose | 0,25 g |
| Propylenglycol | 1,5 g |
| Tween-20-Lösung 1 %-ig | 1,0 g |
| dest. Wasser zu | 10,0 g |

### A. Endodontische Erkrankungen

### A.1. Topische Behandlung von Pulpitiden auf Grund von kariösen Erkrankungen

**Patient A 1.1** (männlich, 43 Jahre). Klinische Diagnose: Zahn 24, kariöser mesialer Defekt (Schmelz/Dentin), Zahn vital, verstärkt Schmerzen auf kalt. Behandlungsverlauf: Entfernung der Karies. Restdentindecke geringfügig erweicht. Pelleteinlage getränkt mit Moxifloxacin-Hydrochlorid-Gel (25 mg/mL), provisorischer Kavitätenverschluss. Kontrolluntersuchung nach 4 Tagen: Patient war beschwerdefrei. Unterfüllung mit Dropsin, Abschlussfüllung mit Composite. Kontrolluntersuchung nach 3 Wochen: Zahn vital, Patient beschwerdefrei.

**Patient A 1.2** (männlich, 33 Jahre). Klinische Diagnose: Zähne 11, 13, größere distale und mesiale kariöse Defekte (Schmelz/Dentin), alle Zähne vital, Zahn 11 mit Schmerzen auf kalt, Zahn 13 mit leichten Beschwerden auf warm. Behandlungsverlauf: Entfernung der Karies. Restdentindecke hart. Pelleteinlagen mit Moxifloxacin-Hydrochlorid-Gel (50 mg/mL), provisorischer Kavitätenverschluß. Kontrolluntersuchung nach 1 Woche: Zähne 11 und 13 beschwerdefrei. Unterfüllung mit Dropsin, Abschlüßfüllung mit Composite Kontrolluntersuchung nach 14 Tagen: Zähne vital, Patient beschwerdefrei.

**Patient A 1.3** (weiblich, 18 Jahre). Klinische Diagnose: Zahn 24, großer distaler kariöser Defekt (Schmelz/Dentin), Zahn vital, Schmerzen auf kalt, süß und sauer. Behandlungsverlauf: Entfernung der Karies. Restdentindecke hart. Pelleteinlage mit Moxifloxacin-Hydrochlorid-Gel (25mg/mL), provisorischer Kavitätenverschluß. Kontrolluntersuchung nach einer Woche: noch unklare, geringe Beschwerden, Wiederholung der Einlage. Kontrolluntersuchung nach 3 Tagen: Zahn vital, Patient beschwerdefrei. Unterfüllung mit Dropsin, Abschlußfüllung Glasionomer.

**Patient A 1.4** (männlich, 23 Jahre). Klinische Diagnose: Zahn 11, großer kariöser mesialer Defekt, Zahn vital, Schmerzen auf warm und kalt, Nachtschmerz. Behandlungsverlauf: Entfernung der Karies. Restdentindecke leicht erweicht. Pelleteinlagen mit Moxifloxacin-Hydrochlorid-Gel (50mg/mL), provisorischer Kavitätenverschluß. Kontrolluntersuchung nach 7 Tagen: Patient beschwerdefrei, Zahn vital. Unterfüllung Zinkphosphatzement, Abschlußfüllung Glasionomer.

**Patient A 1.5** (männlich, 43 Jahre). Klinische Diagnose: Zahn 13, großer distaler kariöser Defekt, Zahn schwach vital, Schmerzen auf kalt, Nachtschmerz. Behandlungsverlauf: Entfernung der Karies. Restdentindecke hart. Pelleteinlage mit Moxifloxacin-Hydrochlorid-Gel (50 mg/mL), provisorischer Kavitätenverschluß. Kontrolluntersuchung nach 4 Tagen: Patient beschwerdefrei, Zahn vital. Unterfüllung Dropsin, Abschlußfüllung Composite.

**Patient A 1.6** (weiblich, 42 Jahre). Klinische Diagnose: Zahn 36, großer mesialer kariöser Defekt, Zahn mit verringerter Vitalität, Schmerzen auf kalt und warm, Nachtschmerz. Behandlungsverlauf: Entfernung der Karies, Pelleteinlage mit Moxifloxacin-Hydrochlorid-Gel (50 mg/mL), provisorischer Kavitätenverschluß. Kontrolluntersuchung nach 6 Tagen: noch geringe Schmerzen auf warm und kalt. Wiederholung der Einlage. Kontrolluntersuchung nach 7 Tagen: Patient beschwerdefrei, Zahn vital. Unterfüllung Zinkphosphatzement, Abschlußfüllung Composite.

**Patient A 1.7** (weiblich, 18 Jahre). Klinische Diagnose: Zahn 44, großer mesialer kariöser Defekt, Zahn vermindert vital, Schmerzen auf kalt, Nachtschmerz. Behandlungsverlauf: Entfernung der Karies. Restdentindecke leicht erweicht. Pelleteinlagen mit Moxifloxacin-Hydrochlorid-Gel (50 mg/mL), provisorischer Kavitätenverschluß. Kontrolluntersuchung nach 4 Tagen: Patient beschwerdefrei, Unterfüllung Zinkphosphatzement, Abschlußfüllung Composite.

**Patient A 1.8** (männlich, 38 Jahre). Klinische Diagnose: Zahn 36, großer distaler kariöser Defekt, Zahn vermindert vital, Schmerzen auf kalt, warm, süß, sauer, leicht perkussionsempfindlich, Nachtschmerz. Behandlungverlauf: Entfernung der Karies. Restdentindecke leicht erweicht. Pelleteinlage mit Moxifloxacin-Hydrochlorid-Gel (50 mg/mL), provisorischer Kavitätenverschluß.

Kontrolluntersuchung nach 3 Wochen: Patient beschwerdefrei, Zahn vital, Unterfüllung Zinkphosphat, Abschlußfüllung Composite.

**Patient A 1.9** (weiblich, 50 Jahre). Klinische Diagnose: Zahn 27, großer distaler kariöser Defekt, Zahn vital, Beschwerden auf kalt, leichte Beschwerden auf warm, leichter Nachtschmerz. Behandiungsverlauf: Entfernung der Karies. Restdentindecke erweicht. Pelleteinlage mit Moxifloxacin-Hydrochlorid-Gel (25 mg/mL), provisorischer Verschluß. Kontolluntersuchung nach 7 Tagen: Patientin beschwerdefrei, Zahn vital.

**Patient A 1.10** (männlich, 17 Jahre). Klinische Diagnose: Zähne 16, 17, 27, 36, 37, 46 okklusal kariös, Zähne vital, Schmerzen auf kalt, süß und sauer, manchmal auf warm, nicht lokalisierbar. Behandlungsverlauf: Entfernung der Karies. Restdentindecken hart. Pelleteinlagen mit Moxifloxacin-Hydrochlorid-Gel (50mg/mL), provisorischer Kavitätenverschluß. Kontrolluntersuchungen nach 14 Tagen: Patient beschwerdefrei, Zähne vital. Unterfüllung Dropsin, Abschlußfüllung Composite.

**Patient A 1.11** (männlich, 33 Jahre). Klinische Diagnose: Zahn 22, kariöser distaler Defekt (Schmelz/Dentin), Zahn vital, leichte Schmerzen auf warm. Behandlungsverlauf: Entfernung der Karies, Restdentindecke geringfügig erweicht. Pelleteinlage getränkt mit Grepafloxacin-Gel (25 mg/mL), provisorischer Kavitätenverschluß. Kontrolluntersuchung nach 5 Tagen: Patient war beschwerdefrei. Unterfüllung mit Zink-Phosphatzement, Abschlußfüllung mit Composite. Kontrolluntersuchung nach 4 Wochen: Zahn vital, Patient beschwerdefrei.

**Patient A 1.12** (männlich, 38 Jahre). Klinische Diagnose: Zähne 22, 23 mit größeren distalen kariösen Defekten (Schmelz/Dentin), Zähne vital, Schmerzen auf kalt. Behandlungsverlauf: Entfernung der Karies, Restdentindecke hart. Pelleteinlagen mit Grepafloxacin-Gel (50 mg/mL), provisorischer Kavitätenverschluß. Kontrolluntersuchung nach 1 Woche: Zähne beschwerdefrei. Unterfüllung mit Dropsin, Abschlußfüllung mit Composite. Kontrolluntersuchung nach 14 Tagen: Zähne vital, Patient beschwerdefrei.

**Patient A 1.13** (weiblich, 48 Jahre). Klinische Diagnose: Zahn 14, großer mesialer kariöser Defekt (Schmelz/Dentin), Zahn vital, Schmerzen auf kalt und sauer. Beharidlungsveriauf: Entfernung der Karies, Restdentindecke hart. Pelleteinlage mit Gemifloxacin-Mesylat-Gel (25 mg/mL), provisorischer Kavitätenverschluß. Kontrolluntersuchung nach 3 Wochen: noch geringe Beschwerden. Wiederholung der Einlage. Kontrolluntersuchung nach 4 Tagen: Zahn vital, Patient beschwerdefrei. Unterfüllung mit Dropsin, Abschlussfüllung mit Glasionomer.

**Patient A 1.14** (männlich, 53 Jahre). Klinische Diagnose: Zahn 13, großer kariöser mesialer Defekt, Zahn vital, Schmerzen auf warm, Nachtschmerz. Behandlungsverlauf: Entfernung der Karies, Restdentindecke leicht erweicht. Pelleteinlagen mit Levofloxacin-Gel (50 mg/mL), provisorischer Kavitätenverschluß. Kontrolluntersuchung nach 14 Tagen: Patient beschwerdefrei, Zahn vital. Unterfüllung mit Dropsin, Abschlussfüllung mit Composite

**Patient A 1.15** (männlich, 22 Jahre). Klinische Diagnose: Zahn 44, großer mesialer kariöser Defekt, Zahn vital, Schmerzen auf kalt, Nachtschmerz. Behandlungsverlauf: Entfernung der Karies, Restdentindecke lederartig. Pelleteinlage mit Trovafloxacin-Mesylat-Gel (50 mg/mL), provisorischer Kavitätenverschluß. Kontrolluntersuchung nach 9 Tagen: Patient beschwerdefrei, Zahn vital. Unterfüllung mit Dropsin, Abschlussfüllung mit Composite.

**Patient A 1.16** (weiblich, 15 Jahre). Klinische Diagnose: Zahn 46, großer distaler kariöser Defekt, Zahn mit verringerter Vitalität, Schmerzen auf kalt und warm. Behandlungsverlauf: Entfernung der Karies, Pelleteinlage mit Sparfloxacin-Gel (25 mg/mL), provisorischer Kavitätenverschluß. Kontrolluntersuchung nach 4 Tagen: noch geringe Schmerzen auf warm. Wiederholung der Einlage. Kontrolluntersuchung nach 14 Tagen: Patientin beschwerdefrei, Zahn vital. Unterfüllung mit Dropsin, Abschlussfüllung mit Composite.

### A.2. Prophylaxe von Dentinwunden

**Patient A 2.1** (männlich, 30 Jahre). Klinische Diagnose: Zähne 33 und 36, kariöse Defekte an beiden Zähnen. Zähne vital. Behandlungsverlauf: Präparation der Zähne 33 und 36. Anfertigung einer provisorischen Brücke. Auftrag von Moxiftoxacin-Hydrochlorid-Lösung (25 mg/mL) auf Kavität, Trocknung im leichten Luftstrom. Befestigung der Brücke mit provisorischem Zement. Nach 8 Tagen definitives Einsetzen der Brücke mit Zink-Phosphatzement. Kontrolluntersuchung nach 3 Wochen: Patient beschwerdefrei.

**Patient A 2.2** (männlich, 48 Jahre). Klinische Diagnose: Zähne 22, 23, 24 mit kleinen distalen kariösen Defekten (Schmelz/Dentin), alle Zähne vital, Schmerzen auf kalt. Behandlungsverlauf: Entfernung der Karies, Vorstrich mit Moxifloxacin-Hydrochlorid-Lösung (50 mg/mL), provisorischer Kavitätenverschluß. Kontrolluntersuchung nach 2 Wochen: Zähne beschwerdefrei. Unterfüllung mit Dropsin, Abschlußfüllung mit Composite. Kontrolluntersuchung nach 4 Wochen: Zähne vital, Patient beschwerdefrei.

**Patient A 2.3** (weiblich, 28 Jahre). Klinische Diagnose: Zähne 14, 15, 16, 17 mit cervikalen Erosionen (Schmelz/Dentin), Schmerzen auf kalt und süß. Behandlungsverlauf: Präparation, Vorstrich mit Moxifloxacin-Hydrochlorid-Lösung (50 mg/mL), sanfte Trocknung im Luftstrom. Adhäsive Restauration mit Composite. Kontrolluntersuchung nach 3 Wochen: Patientin beschwerdefrei.

**Patient A 2.4** (männlich, 33 Jahre). Klinische Diagnose: Zähne 11, 12, 21, 22 mit kariösen Defekten im ersten Dentindrittel, Zähne vital. Behandlungsverlauf: Präparation für Keramikkronen. Vorstrich mit Moxifloxacin-Hydrochlorid-Lösung (25 mg/mL), Trocknung im sanften Luftstrom, Einsetzen von provisorischen Kronen. Nach 14 Tagen Eingliedern der definitiven Kronen mit Zinkphosphatzement. Kontrolluntersuchung nach 3 Wochen: Patient beschwerdefrei.

**Patient A 2.5** (männlich, 62 Jahre). Klinische Diagnose: Zahn 44, mesialer kariöser Defekt im ersten Dentindrittel, Schmerzen auf kalt. Behandlungsverlauf: Entfernung der Karies, Vorstrich mit Moxifloxacin-Hydrochlorid-Lösung (25 mg/mL), provisorischer Kavitätenverschluß. Kontrolluntersuchung nach 9 Tagen: Patient beschwerdefrei. Unterfüllung Dropsin, Abschlußfüllung Composite.

**Patient A 2.6** (weiblich, 18 Jahre). Klinische Diagnose: Zahn 43, distaler kariöser Defekt im ersten Dentindrittel. Behandlungsverlauf: Entfernung der Karies, Vorstrich mit Moxifloxacin-Hydrochlorid-Lösung (25 mg/mL), Unterfüllung Dropsin, Abschlußfüllung Composite. Patient zur Kontrolluntersuchung nicht erschienen.

**Patient A 2.7** (weiblich, 28 Jahre). Klinische Diagnose: Zähne 15, 14, 13, 12, 11, 21, 22, 23, 24, 25 mit cervikalen Erosionen, starke Hypersensibilität. Behandlungsverlauf. Reinigung der Erosionsbereiche, Vorstrich mit Sparfloxacin-Lösung (50 mg/mL), sanfte Trocknung im Luftstrom. Adhäsive Restauration mit Composite.

**Patient A 2.8** (weiblich, 33 Jahre). Klinische Diagnose: Zähne 13, 14, 15 mit kariösen Defekten mesial und distal im ersten Dentindrittel, Schmerzen auf kalt. Behandlungsverlauf: Präparation der Kavitäten, Entfernung der Karies, Vorstrich mit Trovafloxacin-Mesylat-Lösung (25 mg/mL), Trocknung im sanften Luftstrom. Adhäsive Restauration mit Glasionomerzement.

### A.3. Topische Behandlung des infizierten Wurzelkanals und des periapikalen Bereichs

**Patient A 3.1** (weiblich, 40 Jahre). Klinische Diagnose: Zahn 45, apikale Parodontitis basierend auf Pulpitis purulenta, leichte Aufbißbeschwerden. Röntgenologisch leicht erweiterter Parodontalspalt, nicht gangränös. Behandlungsverlauf: Kanalaufbereitung bis zum Apex. Kanaleinlage mit Moxifloxacin-Hydrochlorid-Lösung (100 mg/mL) mittels Spritze, anschließend Nachfüllen von Moxifloxacin-Hydrochlorid-Gel (100 mg/mL), provisorischer Verschluß. Kontrolluntersuchung nach 3 Tagen: Patientin beschwerdefrei, Wurzelfüllung mit Endomethasone. Kontrolluntersuchung nach 14 Tagen: Patientin weiterhin beschwerdefrei, Abschlußfüllung Composite.

**Patient A 3.2** (männlich, 70 Jahre). Klinische Diagnose: Zahn 23, gangränös, Aufbißbeschwerden. Röntgenologisch kein Befund. Behandlungsverlauf: Kanalaufbereitung bis zum Apex, Kanaleinlage mit Moxifloxacin-Hydrochlorid-Lösung (100 mg/mL) mittels Spritze, anschließend Nachfüllen von Moxifloxacin-Hydrochlorid-Gel (100 mg/mL), provisorischer Verschluß. Kontrolluntersuchung nach 14 Tagen: Patient beschwerdefrei, Kanal geruchlos. Wurzelfüllung mit Endomethasone.

**Patient A 3.3** (weiblich, 22 Jahre). Klinische Diagnose: Zahn 11, apikale Parodontitis basierend auf Gangrän, Aufbißbeschwerden. Röntgenologisch erweiterter Parodontalspalt. Behandlungsverlauf: Kanalaufbereitung bis zum Apex, Kanaleinlage mit Moxifloxacin-Hydrochlorid-Lösung (50 mg/mL) mittels Spritze, anschließend Nachfüllen von Moxifloxacin-Hydrochlorid-Gel (50 mg/mL) und intraligamentale Injektion von Moxifloxacin-Hydrochtorid-Lösung (50 mg/mL). Provisorischer Verschluß. Kontrolluntersuchung nach 3 Tagen: Patientin beschwerdefrei, Kanal geruchlos.

**Patient A 3.4** (männlich, 40 Jahre). Klinische Diagnose: Zahn 11, apikate Parodontotis basierend Pulpitis purulenta, nicht gangränös, Aufbißbeschwerden. Röntgenologisch erweiterter Parodontalspalt. Behandlungsverlauf: Kanalaufbereitung bis zum Apex, Wurzelkanaleinlage mit Moxifloxacin-Hydrochlorid-Gel (50 mg/mL), provisorischer Verschluß. Kontrolluntersuchung nach 3 Tagen: Patient beschwerdefrei. Wurzelfüllung mit Endomethasone, provisorischer Verschluß. Kontrolluntersuchung nach 3 Wochen: Patient weiterhin beschwerdefrei. Abschlußfüllung Compsite.

**Patient A 3.5** (weiblich, 43 Jahre). Klinische Diagnose: Zahn 35, apikale Parodontitis basierend auf Gangrän,.leichte Aufbißbeschwerden. Röntgenologisch schwache Aufhellung sichtbar. Behandlungsverlauf: Kanalaufbereitung bis zum Apex nicht möglich. Wurzelkanaleinlage mit Moxifloxacin-Hydrochlorid-Lösung (50 mg/mL) mittels Spritze, anschließend Nachfüllen von Moxifloxacin-Hydrochlorid-Gel (50 mg/mL) und intraligamentale Injektion von Moxifloxacin-Hydrochlorid-Lösung (50 mg/mL). Kontrolluntersuchung nach 7 Tagen: kein gangränöser Geruch, keine Aufbißbeschwerden. Wurzelfüllung mit N2 medical. Kontrolluntersuchung nach 7 Tagen: Patientin beschwerdefrei. Abschlußfüllung mit Composite

**Patient A 3.6** (männlich, 29 Jahre). Klinische Diagnose: Zahn 36, apikale Parodontitis basierend auf Gangrän, Aufbißbeschwerden. Röntgenologisch kein Befund. Behandlungsverlauf: Kanalaufbereitung bis zum Apex, Wurzelkanaleinlage mit Moxifloxacin-Hydrochlorid-Gel (50 mg/mL) und intraligamentale Injektion von Moxifloxacin-Hydrochlorid-Lösung (50 mg/mL). Kontrolluntersuchung nach 8 Tagen: kein gangränöser Geruch, Patient beschwerdefrei. Wurzelfüllung mit N2 medical. Kontrolluntersuchung nach 3 Wochen: Patient beschwerdefrei. Abschlußfüllung mit Composite.

**Patient A 3.7** (weiblich, 50 Jahre). Klinische Diagnose: Zahn 14, apikale Parodontitis basierend auf Gangrän. Geringe Aufbißbeschwerden. Röntgenologisch schwache Aufhellung. Behandlungsverlauf: Kanalaufbereitung bis zum Apex, Wurzelkanaleinlage mit Moxifloxacin-Hydrochlorid-Gel (100 mg/mL) und intraligamentale Injektion von Moxifloxacin-Hydrochtorid-Lösung (50 mg/mL), provisorischer Verschluß. Kontrolluntersuchung nach 7 Tagen: Patientin beschwerdefrei. Wurzelfüllung mit Endomethasone. Kontrolluntersuchung nach 3 Wochen: Patientin weiterhin beschwerdefrei. Abschlußfüllung mit Composite.

**Patient A 3.8** (weiblich, 42 Jahre). Klinische Diagnose: Zahn 34, apikale Parodontitis basierend auf Gangrän, Aufbißbeschwerden. Röntgenologisch schwache Aufhellung sichtbar. Behandlungsverlauf: Kanalaufbereitung bis zum Apex nicht möglich. Wurzelkanaleinlage mit Moxifloxacin-Hydrochlorid-Gel (50 mg/mL) und intraligamentale Injektion von Moxifloxacin-Hydrochlorid-Lösung (50 mg/mL), provisorischer Verschluß. Kontrolluntersuchung nach 13 Wochen: Patientin beschwerdefrei, Kanal geruchsfrei. Wurzelfüllung mit Endomethasone, Abschlußfüllung mit Glasionomer.

**Patient A 3.9** (weiblich, 17 Jahre). Klinische Diagnose: Zahn 46, apikale Parodontitis basierend auf Pulpitis purulenta, leichte Aufbißbeschwerden, Zahn nicht gangränös. Röntgenologisch leicht erweiterter Parodontalspalt an mesialer Wurzel. Behandlungsverlauf: Kanäle aufbereitet bis zum Apex, Wurzelkanaleinlage mit Moxifloxacin-Hydrochlorid-Gel (100 mg/mL) und intraligamentale Injektion von Moxifloxacin-Hydrochlorid-Lösung (50 mg/mL). Kontrolluntersuchung nach 7 Tagen: Patientin beschwerdefrei. Wurzelfüllung mit Endomethasone, Abschlußfüllung. Patientin zur weiteren Kontrolluntersuchung nicht erschienen.

**Patient A 3.10** (weiblich, 24 Jahre). Klinische Diagnose: Zahn 14, Fistel im Wurzelspitzenbereich, Zahn gangränös. Leichte Aufbißbeschwerden. Behandlungsverlauf: Kanalaufbereitung bis zum Apex. Mit Moxifloxacin-Hydrochlorid-Lösung (50 mg/mL) mittels Spritze Kanal und Fistelgang durchspült, Kanaleinlage mit Moxifloxacin-Hydrochlorid-Gel (50 mg/mL) und intraligamentale Injektion von Moxifloxacin-Hydrochlorid-Lösung (50 mg/mL). Kanal nur mit Wattebausch verschlossen. Kontrolluntersuchung nach 3 Tagen: Patientin noch nicht völlig beschwerdefrei. Kanal zeigte noch geringfügigen gangränösen Geruch. Wiederholung der Durchspülung mit Moxifloxacin-Hydrochlorid-Lösung (50 mg/mL) und der Kanaleinlage mit Moxifloxacin-Hydrochlorid-Gel (50 mg/mL). Kontrolluntersuchung nach 8 Tagen: Patientin beschwerdefrei. Wurzelfüllung mit Endomethasone. Kontrolluntersuchung nach 3 Wochen: Patientin beschwerdefrei. Abschlußfüllung mit Composite.

**Patient A 3.11** (männlich, 51 Jahre). Klinische Diagnose: Zahn 34, apikale Parodontitis basierend auf Pulpitis purulenta, nicht gangränös, keine Aufbißbeschwerden. Fistel im Wurzelspitzenbereich. Röntgenologisch leichte Aufhellung. Behandlungsverlauf: Kanalaufbereitung bis zum Apex nicht möglich an palatinaler Wurzel. Wurzelkanaleinlage mit Moxifloxacin-Hydrochlorid-Gel (100 mg/mL), provisorischer Verschluß. Kontrolluntersuchung nach 10 Tagen: Patient beschwerdefrei. Wurzelfüllung mit Endomethasone. Patient zur Kontrolluntersuchung nicht erschienen.

**Patient A 3.12** (weiblich, 34 Jahre). Klinische Diagnose: Zahn 44, Fistelbildung, Aufbißschwierigkeiten, gangränös. Behandlungsverlauf: Kanalaufbereitung bis zum Apex. Wurzelkanaleinlage mit Moxiftoxacin-Hydrochtorid-Lösung (50 mg/mL) mit Hilfe einer Papierspitze und intraligamentale Injektion von Moxifloxacin-Hydrochlorid-Lösung (50 mg/mL), provisorischer Verschluß. Kontrolluntersuchung nach 8 Tagen: Patientin beschwerdefrei. Wurzelfüllung mit Endomethasone, Abschlußfüllung mit Composite.

**Patient A 3.13** (weiblich, 78 Jahre). Klinische Diagnose: Zahn 15, apikale Parodontitis basierend auf Pulpitis purulenta, leichte Aufbissbeschwerden, nicht gangränös. Behandlungsverlauf: Kanalaufbereitung bis zum Apex, Kanaleinlage mit Moxifloxacin-Hydrochlorid-Lösung (50 mg/mL) mittels Spritze und anschließend Nachfüllen von Moxifloxacin-Hydrochlorid-Gel (50 mg/mL) und intraligamentale Injektion von Moxifloxacin-Hydrochlorid-Lösung (50 mg/mL), provisorischer Verschluss. Kontrolluntersuchung nach 9 Tagen: Patientin beschwerdefrei, Wurzelfüllung mit Endomethasone. Kontrolluntersuchung nach 10 Tagen: Patientin weiterhin beschwerdefrei, Abschlußfüllung Composite.

**Patient A 3.14** (männlich, 30 Jahre). Klinische Diagnose: Zahn 13, gangränös, Aufbissbeschwerden, röntgenologisch kein Befund. Behandlungsverlauf: Kanalaufbereitung bis zum Apex; Kanaleinlage mit Moxifloxacin-Hydrochlorid-Lösung (50 mg/mL) mittels Spritze und anschließend Nachfüllen von Moxifloxacin-Hydrochlorid-Gel (50 mg/mL) und intraligamentale Injektion von Moxifloxacin-Hydrochlorid-Lösung (50 mg/mL), provisorischer Verschluss. Kontrolluntersuchung nach 10 Tagen: Patient beschwerdefrei, Kanal geruchlos, Wurzelfüllung mit Endomethasone.

**Patient A 3.15** (weiblich, 32 Jahre). Klinische Diagnose: Zahn 31, apikale Parodontitis basierend auf Gangrän, Aufbissbeschwerden, röntgenologisch erweiterter Parodontalspalt. Behandlungsverlauf: Kanalaufbereitung bis zum Apex, Kanaleinlage mit Moxifloxacin-Hydrochlorid-Lösung (50 mg/mL) mittels Spritze, anschließend Nachfüllen von Moxifloxacin-Hydrochlorid-Gel (50 mg/mL) und intraligamentale Injektion von Moxifloxacin-Hydrochlorid-Lösung (50 mg/mL), provisorischer Verschluss. Kontrolluntersuchung nach 9 Tagen: Patientin beschwerdefrei, Kanal geruchlos.

**Patient A 3.16** (männlich, 40 Jahre). Klinische Diagnose: Zahn 46, apikale Parodontotis, basierend Pulpitis purulenta, nicht gangränös, röntgenologisch erweiterter Parodontalspalt sichtbar. Behandlungsverlauf: Kanalaufbereitung bis zum Apex, Wurzelkanaleinlage mit Grepafloxacin-Gel (50 mg/mL), provisorischer Verschluss. Kontrolluntersuchung nach 3 Wochen: Patient beschwerdefrei. Wurzelfüllung mit Endomethasone, provisorischer Verschluss. Kontrolluntersuchung nach 1 Woche: Patient weiterhin beschwerdefrei, Abschlussfüllung Composite.

**Patient A 3.17** (weiblich, 33 Jahre). Klinische Diagnose: Zahn 32, apikale Parodontitis basierend auf Gangrän, leichte Aufbissbeschwerden, röntgenologisch schwache Aufhellung sichtbar. Behandlungsverlauf: Kanalaufbereitung bis zum Apex nicht möglich. Wurzelkanaleinlage mit Grepafloxacin-Lösung (20 mg/mL) mittels Spritze, anschließend Nachfüllen von Grepafloxacin-Gel (20 mg/mL) und intraligamentale Injektion von Grepafloxacin-Lösung (50 mg/mL). Kontrolluntersuchung nach 6 Tagen: kein gangränöser Geruch, keine Aufbissbeschwerden. Wurzeifüllung mit N2 medical.Kontrolluntersuchung nach 4 Tagen: Patientin beschwerdefrei. Abschlussfüllung mit Composite.

**Patient A 3.18** (weiblich, 55 Jahre). Klinische Diagnose: Zahn 34, apikale Parodontitis basierend auf Gangrän, geringe Aufbissbeschwerden, röntgenologisch schwache Aufhellung sichtbar. Behandlungsverlauf: Kanalaufbereitung bis zum Apex, Wurzelkanaleinlage mit Trovaftoxacin-Mesylat-Gel (100 mg/mL) und intraligamentale Injektion von Trovafloxacin-Mesylat-Lösung (50 mg/mL), provisorischer Verschluss. Kontrolluntersuchung nach 12 Tagen: Patientin beschwerdefrei. Wurzelfüllung mit Endomethasone. Kontrolluntersuchung nach 3 Wochen: Patientin weiterhin beschwerdefrei. Abschlussfüllung mit Composite.

**Patient A 3.19** (weiblich, 22 Jahre). Klinische Diagnose: Zahn 11, apikale Parodontitis basierend auf Gangräna simplex. Behandlungsverlauf: Kanalaufbereitung bis zum Apex nicht möglich. Wurzelkanaleinlage mit Trovafloxacin-Mesylat-Lösung (50 mg/mL), provisorischer Verschluss. Kontrolluntersuchung nach 3 Wochen: Patientin beschwerdefrei, Kanal geruchsfrei. Wurzelfüllung mit Endomethasone, Abschlußfüllung mit Glasionomer.

**Patient A 3.20** (weiblich, 77 Jahre). Klinische Diagnose: Zahn 47, apikale Parodontitis basierend auf Pulpitis purulenta, Zahn nicht gangränös, röntgenologisch leicht erweiterter Parodontalspalt an mesialer Wurzel sichtbar. Behandlungsverlauf: Kanäle aufbereitet bis zum Apex. Wurzelkanaleinlage mit Tosufloxacin-Tosylat-Gel (100 mg/mL). Kontrolluntersuchung nach 8 Tagen: Patientin beschwerdefrei. Wurzelfüllung mit Endomethasone, Abschlußfüllung. Patientin zur weiteren Kontrolluntersuchung nicht erschienen.

**Patient A 3.21** (weiblich, 54 Jahre). Klinische Diagnose: Zahn 22, Fistel im Wurzelspitzenbereich, Zahn gangränös, leichte Aufbissbeschwerden. Behandlungsverlauf: Kanalaufbereitung bis zum Apex, Kanal und Fistelgang mit Moxiftoxacin-Hydrochtorid-Lösung (50 mg/mL) mittels Spritze durchspült, Kanaleinlage mit Moxifloxacin-Hydrochlorid-Gel (50 mg/mL) und intraligamentale Injektion von Moxifloxacin-Hydrochlorid-Lösung (50 mg/mL). Kanal nur mit Wattebausch verschlossen. Kontrolluntersuchung nach 8 Tagen: Patientin noch nicht völlig beschwerdefrei, Kanal zeigte noch geringfügigen Geruch. Wiederholung der Durchspülung und der Einlage. Kontrolluntersuchung nach 10 Tagen: Patientin beschwerdefrei. Wurzelfüllung mit Endomethasone. Kontrolluntersuchung nach 1 Woche: Patientin beschwerdefrei. Abschlussfüllung mit Composite.

**Patient A 3.22** (männlich, 50 Jahre). Klinische Diagnose: Zahn 35, apikale Parodontitis basierend auf Pulpitis purulenta, nicht gangränös, keine Aufbissbeschwerden. Behandlungsverlauf: Kanalaufbereitung bis zum Apex, Wurzelkanaleinlage mit Tosufloxacin-Tosylat-Gel (50 mg/mL), provisorischer Verschluss.Kontrolluntersuchung nach 10 Tagen: Patient beschwerdefrei. Wurzelfüllung mit Endomethasone. Patient zur Kontrolluntersuchung nicht erschienen.

**Patient A 3.23** (weiblich, 64 Jahre). Klinische Diagnose: Zahn 34, Fistelbildung, Aufbissschwierigkeiten, gangränös. Behandlungsverlauf: Kanalaufbereitung bis zum Apex, Wurzelkanaleinlage mit Grepafloxacin-Lösung (50 mg/mL) mit Hilfe einer Papierspitze, intraligamentale Injektion von Grepafloxacin-Lösung (50 mg/mL), provisorischer Verschluss. Kontrolluntersuchung nach 10 Tagen: Patientin beschwerdefrei. Wurzelfüllung mit Endomethasone, Abschlussfüllung mit Composite.

### B Topische Behandlung von Parodontopathien

**Patient B 1** (weiblich, 33 Jahre). Klinische Diagnostik: Zähne 26 und 27, marginale Parodontitis mit freiliegendem Wurzelzement, Taschentiefen von 4 bis 5 mm, starker foetor ex ore. Behandlungsverlauf: Konkremententfemung mittels Ultraschall und manuell, Fadeneinlage mit Moxifloxacin-Hydrochlorid-Gel (50 mg/mL). Abdeckung mit Zahnfleischverband Gingipac. Kontrolluntersuchung nach 3 Tagen: Patientin beschwerdefrei, Gingivalbereiche und Taschen entzündurigsfrei.

**Patient B 2** (weiblich, 60 Jahre). Klinische Diagnose: Zähne 14 bis 18, starke marginale Parodontitis mit freiliegendem Wurzelzement, Taschentiefen 5 bis 6 mm. Behandlungsverlauf: Konkremententfemung mit Ultraschall, Fadeneinlage mit Moxifloxacin-Hydrochlorid-Gel (50 mg/mL), Abdeckung mit Zahniteischverband Gingipac. Kontrolluntersuchung nach 4 Tagen: Patientin beschwerdefrei, Gingivalbereiche und Taschen entzündungsfrei.

**Patient B 3** (männlich, 49 Jahre alt). Klinische Diagnose: Zähne 24 bis 28, marginale Parodontitis mit freiliegendem Wurzelzement, Taschentiefen 4 bis 6 mm. Behandlungsverlauf: Konkremententfemung mit Ultraschall und manuell, Fadeneinlage mit Moxifloxacin-Hydrochlorid (50 mg/mL), Abdeckung mit Zahnfleischverband Gingipac. Kontroltuntersuchung nach 5 Tagen: Patient beschwerdefrei, Gingivalbereiche und Taschen entzündungsfrei.

**Patient B 4** (weiblich, 20 Jahre). Klinische Diagnose: Gesamter Ober- und Unterkieferbereich marginale Parodontitis, starker foetor ex ore, Taschentiefen 4 bis 5 mm. Behandlungsverlauf: Konkremententfemung mit Ultraschall, Medikamentenschiene über 4 Tage für je 10 Minuten mit Moxifloxacin-Hydrochlorid-Gel (0,25 mg/mL) getragen. Kontrolluntersuchung nach 3 Tagen: Patientin beschwerdefrei, keine Entzündungserscheinungen mehr vorhanden.

**Patient B 5** (weiblich, 53 Jahre). Klinische Diagnostik: Zahn 37, marginale Parodontitis mit freiliegendem Wurzelzement, Taschentiefen von 5 mm, starker foetor ex ore. Behandlungsverlauf: Konkremententfemung mittels Ultraschall und manuell, Fadeneinlage mit Moxifioxacin-Hydrochlorid-Gel (50 mg/mL), Abdeckung mit Zahnfleischverband Gingipac. Kontrolluntersuchung nach 8 Tagen: Patientin beschwerdefrei, Gingivalbereiche und Taschen entzündungsfrei.

**Patient B 6** (weiblich, 30 Jahre). Klinische Diagnose: Zähne 25 - 28, starke marginale Parodontitis mit freiliegendem Wurzelzement, Taschentiefen 4 mm. Behandlungsverlauf: Konkremententfemung mit Ultraschall, Fadeneinlage mit Moxifloxacin-Hydrochlorid-Gel (50 mg/mL), intraligamentale Injektion von Moxiftoxacin-Hydrochtond-Lösung (50 mg/mL), Abdeckung mit Zahnfleischverband Gingipac. Kontrolluntersuchung nach 10 Tagen: Patientin beschwerdefrei, Gingivalbereiche und Taschen entzündungsfrei.

**Patient B 7** (männlich, 35 Jahre). Klinische Diagnose: Zähne 14 - 17, marginale Parodontitis mit freiliegendem Wurzeizement, Taschentiefen 4 mm. Behandlungsverlauf: Konkremententfemung mit Ultraschall und manuell, Fadeneinlage mit Grepafloxacin-Gel (50 mg/mL), intraligamentale Injektion von Grepafloxacin-Lösung (50 mg/mL), Abdeckung mit Zahnfleischverband Gingipac. Kontrolluntersuchung nach 9 Tagen: Patient beschwerdefrei, Gingivalbereiche und Taschen entzündungsfrei.

**Patient B 8** (weiblich, 33 Jahre). Klinische Diagnose: im gesamten Ober- und Unterkieferbereich marginale Parodontitis, starker foetor ex ore, Taschentiefen 3 - 5 mm. Behandlungsverlauf: Konkremententfernung mit Ultraschall, Medikamentenschiene über 6 Tage für je 10 Minuten mit Trovafloxacin-Mesylat-Gel (0,25 mg/mL) getragen. Kontrolluntersuchung nach 6 Tagen: Patientin beschwerdefrei, keine Entzündungserscheinungen mehr vorhanden.

### C Topische Behandlung von Schleimhaut-Knochenwunden

**Patient C 1** (männlich, 14 Jahre). Klinische Diagnose: Zähne 011 und 012, Wundversorgung nach Extraktion, reimplantierte Zähne. Behandlungsverlauf: Röntgenologische Resorption, Ankylose und Sequester. Nach Wundversorgung Excochleation. Applikation eines Moxifloxacin-Hydrochlorid-Geldepots (50 mg/mL) mittels Gazestreifens. Kontrolluntersuchung nach 3 Tagen: Patient beschwerdefrei, gute Wundrandadaptation.

**Patient C 2** (männlich, 16 Jahre). Klinische Diagnose: Regio 36, Wundversorgung nach größeren chirurgischen Eingriffen, leichte submucöse Schwellung, Fistelbildung nach pulpitis purulenta an Zahn 36. Behandlungsverlauf: Incision, Durchspülung mit Moxifloxacin-Hydrochlorid-Lösung (50 mg/mL), Streifeneinlage mit Moxifloxacin-Hydrochlorid-Gel (50 mgmL). Kontrolluntersuchung nach 3 Tagen: Patient beschwerdefrei, guter und schneller Heilungsverlauf.

**Patient C 3** (weiblich, 34 Jahre). Klinische Diagnose: Zähne 26 und 46 mit apikalen Granulomen, starke Aufbißbeschwerden. Behandlungsverlauf: Extraktion im entzündlichen Stadium (Osteotomie), kein Nahtverschluß. Wundversorgung mit Gaze, getränkt mit Moxifloxacin-Hydrochlorid-Gel (50 mg/mL). Kontrolluntersuchung nach 2 Tagen: Patientin beschwerdefrei.

**Patient C 4** (weiblich, 34 Jahre). Klinische Diagnose: Zahn 48, impaktiert und verlagert. Behandlungsverlauf: Wundversorgung nach größerem chirurgischen Eingriff (Osteotomie), Wundversorgung mit Gaze, getränkt mit Moxifloxacin-Hydrochlorid-Gel (50 mg/mL). Wundverschluß mit zwei Knopfnähten. Kontrolluntersuchung nach 2 Tagen: Leichte Wundschmerzen, kein postoperatives Ödem. Kontrolluntersuchung nach einer Woche: Nahtentfemung, gute Wundrandadaptation, Patientin beschwerdefrei

**Patient C 5** (männlich, 52 Jahre). Klinische Diagnose: Zahn 34, Pulpitis purulenta, Fistelbildung, submucöse Schwellung. Behandlungsverlauf: Trepanation, Incision, Durchspülung von Wurzelkanal und Fistelgang mit Moxifloxacin-Hydrochlorid-Lösung (50 mg/mL), Kanaleinlage mit Moxifloxacin-Hydrochlorid-Gel (50 mg/mL), Abschluß nur mit Wattebausch, Einlage von Gaze in incidiertem Bereich, getränkt mit Moxifloxacin-Hydrochlorid-Gel. (50 mg/mL), kein Nahtverschluß. Kontrolluntersuchung nach 3 Tagen: Guter Heilungsverlauf, Patient beschwerdefrei. Wurzelkanaltüllung mit Endomethasone. Kontrolluntersuchung nach 3 Wochen: Patient beschwerdefrei.

**Patient C 6** (männlich, 44 Jahre). Klinische Diagnose: Zahn 36, Wundversorgung nach Extraktion. Behandlungsvertauf: Dolor post, Auffrischung der Alveole, Applikation eines Depots aus Moxifloxacin-Hydröchlorid-Gel ( 50 mg/mL) mittels Gazestreifen. Kontrolluntersuchung nach 3 Tagen: Patient beschwerdefrei, gute Wundrandadaptation.

**Patient C 7** (männlich, 36 Jahre). Klinische Diagnostik: Zahn 22, Wundversorgung nach Wurzelspitzenresektion auf Grund einer Zyste. Behandlungsverlauf: Incision, Streifeneinlage mit Moxifloxacin-Hydrochlorid-Gel (50 mg/mL). Kontrolluntersuchung nach 3 Tagen: Patient beschwerdefrei, guter Heilungsverlauf.

**Patient C 8** (weiblich, 31 Jahre). Klinische Diagnostik:Zähne 26 und 26 mit apikalen Granulomen, Aufbissbeschwerden. Behandlungsverlauf: Extraktion im entzündlichen Stadium (Osteotomie), Wundversorgung mit Gaze, getränkt mit Grepafloxacin-Gel (50 mg/mL). Kontrolluntersuchung nach 3 Tagen: Patientin ist beschwerdefrei.

**Patient C 9** (männlich, 22 Jahre). Klinische Diagnostik: Zahn 14, Pulpitis purulenta, Fistelbildung, submucöse Schwellung. Behnadlungsverlauf: Trepanation, Incision.Durchspülung von Wurzelkanal und Fistelgang mit Moxifloxacin-Hydrochlorid-Lösung (25 mg/mL), Kanaleinlage mit Grepafloxacin-Gel (25 mg/mL), Abschluss nur mit Wattebausch. Einlage von Gaze in incidiertem Bereich, getränkt mit Moxifloxacin-Hydrochlorid-Gel (25 mg/mL), kein Nahtverschluß. Kontrolluntersuchung nach 6 Tagen: Patient beschwerdefrei. Wurzelkanalfüllung mit Endomethasone. Kontrolluntersuchung nach 2 Wochen: Patient beschwerdefrei.

### D Wundversorgung

**Patient D 1** (männlich, 33 Jahre). Diagnose: Combustio escharotica am linken Unterarm. Behandlungsverlauf: Wundhygiene, Auftragen von Moxiftoxacin-Hydrochlorid-Gel (1 %-ig), Wundabdeckung. Regenierierung des Hautepithels mit vollständiger Schließung der Wunde nach einer Woche.

**Patient D 2** (männlich, 25 Jahre). Diagnose: Panaritium parunguale am rechten Mittelfinger. Behandlungsverlauf: Leitungsanästhesie, Eröffnung am Nagelrand, lokale Antibiose mit Moxifloxacin-Hydrochlorid-Gel (1 %-ig), Einlage einer Gummilasche. Patient beschwerdefrei.

**Patient D 3** (männlich, 70 Jahre). Diagnose: Diabetes mellitus, Krankenhausaufenthalt wegen schwerer diabetischer Mikroangiopathie der Füße mit diabetischem Fußsyndrom. Danach ambulante Versorgung des Fußes. Befund: a) tiefes, 2 x 1,5 cm großes Ulcus unter rechter Großzehe, schmierig; b) großes Ulcus D III, 5 mm Durchmesser; c) großer Riss D IV / D V; starke Verhornung des ganzen Fußes. Behandlungsverlauf: systemischer Behandlungsversuch mit Avalox 400 und Clont 400 ohne nennenswerte Befundänderung. Danach Applikation von Verbänden mit Moxifloxacin-Hydrochlorid-Gel (1 %-ig) in wöchentlichem Abstand. Wundkontrollen nach 1. Woche: a) Ulcus D I 1 x 0,5 cm, sauber; b) Ulcus D III sauber; c) Ulcus D IV / D V verkrustet. Wundkontrollen nach 2. Woche: a) Ulcus D I stecknadelkopfgroß, sauber; b) Ulcus D III sauberes Granulationsgewebe; c) Ulcus C IV / DV abgeheilt. Wundkontrollen nach 3. Woche: a) Ulcus DI unverändert; b) Ulcus D III stecknadelkopfgroß. Wundkontrollen nach 4. Woche: a) Ulcus D I abgeheilt; b) Ulcus D III abgeheilt.

**Patient D 4** (männlich, 62 Jahre). Diagnose: Diabetes mellitus, diabetische Mikroangiopathie des rechten Fußes mit diabetischem Fußsyndrom. Befund: 1 x 1 cm großes Ulcus, schmierig; starke Verhornung des ganzen Fußes. Behandlungsverlauf: Applikation von Verbänden mit Moxifloxacin-Hydrochlorid-Gel (1 %-ig) in wöchentlichem Abstand. Wundkontrolle nach 1. Woche: Ulcus 0,2 x 0,4 cm, sauber. Wundkontrolle nach 2. Woche: Ulcus abgeheilt.

**Patient D 5** (weiblich, 67 Jahre). Diagnose: diabetische Gangrän an der linken Großzehe. Behandlungsverlauf: Wundhygiene, Abtragen von Hyperkeratosen und Nekrosen. Viermaliges Auftragen von Moxifloxacin-Hydrochlorid-Gel (1 %-ig) in wöchentlichem Abstand, jeweils Wundabdeckung. Regenierierung des Hautepithels mit vollständiger Schließung der Wunde, Revaskularisierung.

**Patient D 6** (männlich, 34 Jahre). Diagnose: Erysipel am rechten Unterschenkel. Behandlungsverlauf: Ruhigstellung, lokale Antibiose mit Moxifloxacin-Hydrochlorid-Gel (1 %-ig), lokale Umschläge. Nach 2 Tagen Rezidivprophylaxe. Patient beschwerdefrei.

**Patient D 7** (weiblich, 52 Jahre). Diagnose: Phlegmone an linker Innenhand. Behandlungsverlauf: Incision und breite Eröffnung der betroffenen Gewebsareate, lokale Antibiose mit Moxifloxacin-Hydrochlorid-Gel (1 %-ig), Wundversorgung. Nach 2 Tagen Wiederholung der lokalen Antibiose. Patient beschwerdefrei.

**Patient D 8** (weiblich, 28 Jahre). Diagnose: Furunkel an linkem Unterarm. Behandlungsverlauf: Incision und offene Wundbehandlung mit Moxifloxacin-Hydrochlorid-Lösung (1 %-ig). Patient beschwerdefrei.

**Patient D 9** (weiblich, 44 Jahre). Diagnose: Karbunkel im Nacken. Behandlungsverlauf: Ausschneiden aller nekrotischen Areale, offene Wundbehandlung mit Moxifloxacin-Hydrochlorid-getränktem Umschlag. Patient beschwerdefrei.

## Patentansprüche

1. Verwendung wenigstens einer der Verbindungen:
7-(3-Amino-1-pyrrolidinyl)-8-chlor-1-cyclopropyl-6-fluor-1,4-dihydro-4-oxo-3-chinolincarbonsäure (Clinafloxacin), 1-Cyclopropyl-6-fluor-1,4-dihydro-8-methoxy-7-(3-methyl-1-piperazinyl)-4-oxo-3-chinolincarbonsäure (Gatifloxacin), 7-[(4Z)-3-(Aminomethyl)-4-(methoxyimino)-1-pyrrolidinyl]-1-cyclopropyl-6-fluor-1,4-dihydro-4-oxo-1,8-naphthyridin-3-carbonsäure (Gemifloxacin), 1-Cyclopropyl-6-fluor-1,4-dihydro-5-methyl-7-(3-methyl-1-piperazinyl)-4-oxo-3-chinolincarbonsäure (Grepafloxacin), (3S)-9-Fluor-2,3-dihydro-3-methyl-10-(4-methyl-1-piperazinyl)-7-oxo-7H-pyrido[1,2,3-de]-1,4-benzoxazine-6-carbonsäure (Levofloxacin), 1-Cyclopropyl-6-fluor-1,4-dihydro-8-methoxy-7-[(4aS,7aS)-octahydro-6H-pyrrolo[3,4-b]pyridin-6-yl]-4-oxo-3-chinolincarbonsäure (Moxifloxacin), 5-Amino-1-cyclopropyl-7-[(3R,5S)-3,5-dimethyl-1-piperazinyl]-6,8-difluor-1,4-dihydro-4-oxo-3-chinolincarbonsäure (Sparfloxacin), 7-(3-Amino-1-pyrrotidinyi)-1-(2,4-difluorphenyl)-6-fluor-1,4-dihydro-4-oxo-1,8-naphthyridin-3-carbonsäure (Tosufloxacin) und 7-(1α,5α,6α-6-Amino-3-azabicyclo[3.1.0]hex-3-yl)-1-(2,4-difluorphenyl)-6-fluor-1,4-dihydro-4-oxo-1,8-naphthyridin-3-carbonsäure (Trovafloxacin)
und/oder wenigstens einer Verbindung der allgemeinen Formel (II) 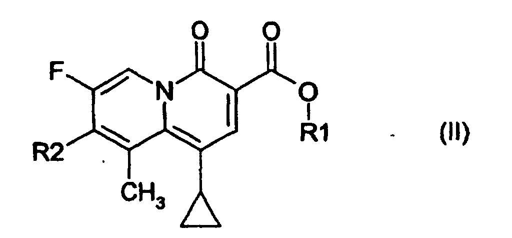 in welcher
R1 für Wasserstoff oder C₁-C₃-Alkyl steht und
R2 für einen gegebenenfalls einfach oder mehrfach substituierten gesättigten oder wenigstens eine Doppelbindung aufweisenden mono-, bi- oder tricyclischen Alicyclus mit gegebenenfalls wenigstens einem Heteroatom im Ringsystem oder einen gegebenenfalls wenigstens ein Heteroatom aufweisenden aromatischen Mono-, Bi- oder Tricyclus steht,
und/oder ihres entsprechenden Hydrates und/oder ihres entsprechenden physiologisch verträglichen Säureadditionssalzes und/oder ggf. ihres entsprechenden physiologisch verträglichen Salzes der den aufgezählten Verbindungen zugrundeliegende Carbonsäure und/oder der zugrundeliegenden Carbonsäure für Verbindungen der allgemeinen Formel (II), worin R1 für H steht, und/oder entsprechender Enantiomere und/oder entsprechender Diastereomere und/oder des entsprechenden Racemates und/oder eines entsprechenden Gemisches aus wenigstens zwei der vorstehend genannten Verbindungen
sowie gegebenenfalls weiterer physiologisch verträglicher Hilfsstoffe zur Herstellung eines Arzneimittels zur lokalen und/oder topischen Behandlung und/ oder zur Prophylaxe von bakteriell verursachten Erkrankungen im Mundbereich und/oder Zahnbereich und/oder Kieferbereich bei Mensch und Tier.

2. Verwendung gemäß Anspruch 1, **dadurch gekennzeichnet, dass** in der allgemeinen Formel (II),
R1 für Wasserstoff steht und
R2 für gegebenenfalls einfach oder mehrfach substituiertes Cyclopropyl, Azetidinyl, Pyrrolidinyl, Piperidinyl, Piperazinyl oder Morpholinyl steht, wobei gegebenenfalls auch wenigstens zwei Substituenten miteinander verknüpft sein können.

3. Verwendung gemäß Anspruch 1 oder 2, **dadurch gekennzeichnet, daß** 7-(3-Amino-1-pyrrolidinyl)-8-chlor-1-cyclopropyl-6-fluor-1,4-dihydro-4-oxo-3-chinolincarbonsäure (Clinafloxacin), 1-Cyclopropyl-6-fluor-1,4-dihydro-8-methoxy-7-(3-methyl-1-piperazinyl)-4-oxo-3-chinolincarbonsäure (Gatifloxacin), 7-[(4Z)-3-(Aminomethyl)-4-(methoxyimino)-1-pyrrolidinyl]-1-cyclopropyl-6-fluor-1,4-dihydro-4-oxo-1,8-naphthyridin-3-carbonsäure (Gemifloxacin), 1-Cyclopropyl-6-fluor-1,4-dihydro-8-methoxy-7-[(4aS,7aS)-octahydro-6H-pyrrolo[3,4-b]pyridin-6-yl]-4-oxo-3-chinolincarbonsäure (Moxifloxacin), 5-Amino-1-cyclopropyl-7-[(3R,5S)-3,5-dimethyl-1-piperazinyl]-6,8-difluor-1,4-dihydro-4-oxo-3-chinolincarbonsäure (Sparfloxacin), 7-(3-Amino-1-pyrrolidinyl)-1-(2,4-difluorphenyl)-6-fluor-1,4-dihydro-4-oxo-1,8-naphthyridin-3-carbonsäure (Tosufloxacin) und 7-(1α,5α,6α-6-Amino-3-azabicyclo[3.1.0]hex-3-yl)-1-(2,4-difluorphenyl)-6-fluor-1,4-dihydro-4-oxo-1,8-naphthyridin-3-carbonsäure (Trovafloxacin) oder ein Gemisch aus wenigstens zwei dieser Verbindungen eingesetzt wird.

4. Verwendung gemäß einem der Ansprüche 1 bis 3, **dadurch gekennzeichnet, daß** 1-Cyclopropyl-6-fluor-1,4-dihydro-8-methoxy-7-(3-methyl-1-piperazinyl)-4-oxo-3-chinolincarbonsäure (Gatifloxacin), 7-[(4Z)-3-(Aminomethyl)-4-(methoxyimino)-1-pyrrolidinyl]-1-cyclopropyl-6-fluor-1,4-dihydro-4-oxo-1,8-naphthyridin-3-carbonsäure (Gemifloxacin) und 1-Cyclopropyl-6-fluor-1,4-dihydro-8-methoxy-7-[(4aS,7aS)-octahydro-6H-pyrrolo[3,4-b]pyridin-6-yl]-4-oxo-3-chinolincarbonsäure (Moxifloxacin) oder ein Gemisch aus wenigstens zwei dieser Verbindungen eingesetzt wird.

5. Verwendung gemäß einem der Ansprüche 1 bis 4, **dadurch gekennzeichnet, dass** die Säureadditionssalze ausgewählt sind aus der Gruppe Hydrochlorid, Hydrobromid, Methansulfonat und Tolylsulfonat.

6. Verwendung gemäß einem der Ansprüche 1 bis 5, **dadurch gekennzeichnet, dass** die physiologisch verträglichen Salze der Carbonsäure ausgewählt sind aus der Gruppe der Alkalisalze, Erdalkalisalze, Ammoniumsalze, Silbersalze und Guanidiniumsalze.

7. Verwendung wenigstens einer der Verbindungen gemäß einem der Ansprüche 1 bis 6 zur Herstellung eines Arzneimittels zur lokalen und/oder topischen Behandlung von endodontischen und parodontischen Erkrankungen.

8. Verwendung gemäß einem der Ansprüche 1 bis 7, **dadurch gekennzeichnet, dass** das Arzneimittel zur lokalen Behandlung von Pulpitiden aufgrund von kariösen Erkrankungen, zur topischen Behandlung des infizierten Wurzelkanals und des periapikalen Gewebes, zur topischen Behandlung von Parodontopathie, zur Behandlung von Schleimhaut-Knochenwunden mit gestörter Wundheilung oder zur Behandlung von Weichteilinfektionen eingesetzt wird.

9. Verwendung wenigstens einer der Verbindungen gemäß einem der Ansprüche 1 bis 6 zur Herstellung eines Arzneimittels zur Prophylaxe von Wunden, vorzugsweise Dentinwunden oder zur perioperativen Prophylaxe.

10. Verwendung gemäß Anspruch 9 zur perioperativen Prophylaxe bei Implantationen, Herzoperationen, Transplantationen, neurochirugischen Operationen, Operationen in stark kontaminiertem Gebiet wie Mundhöhle, Ösophagus, Rektum oder Kolon, oder bei Hysterektomie, Gallenwegsoperationen, bei Operationen von Patienten mit Abwehrschwäche oder bei Amputationen.

11. Verwendung wenigstens einer der Verbindungen gemäß einem der Ansprüche 1 bis 6 zur Herstellung eines Arzneimittels zur topischen und/oder lokalen Behandlung des diabetischen Fuß-Syndroms.

12. Verwendung wenigstens einer der Verbindungen gemäß einem der Ansprüche 1 bis 6 zur Herstellung eines Arzneimittels zur Beschleunigung der Wundheilung.

13. Verwendung gemäß einem der Ansprüche 7 bis 12, **dadurch gekennzeichnet, dass** das Arzneimittel zur Behandlung von Mensch oder Tier eingesetzt wird.

14. Verwendung gemäß einem der Ansprüche 1 bis 13, **dadurch gekennzeichnet, dass** das Arzneimittel in Form eines Gels einer Lösung, einer Suspension, einer Emulsion, in Form von Liposomen oder in Form von Mizellen vorliegt und gegebenenfalls auf ein Trägermaterial oder einen inerten Träger, vorzugsweise eine Streifeneinlage, Fadeneinlage, ein Chip, Tray, Kollagenschwamm, ein Tampon, Wattebauschoder Schaumstoffpellet aufgebracht oder darin eingebracht worden ist.

15. Verwendung gemäß Anspruch 14, **dadurch gekennzeichnet, dass** das Arzneimittel in Form einer wäßrigen Lösung vorliegt.

16. Verwendung gemäß Anspruch 14 oder 15, **dadurch gekennzeichnet, dass** das Arzneimittel als weitere physiologisch verträgliche Hilfsstoffe Lösungsmittel, gelbildner Löslichkeitsverbesserer, Verdickungsmittel, Lösungsvermittler, Solubilisatoren, Konservierungsmittel, Emulgatoren, Schleimstoffe, Osmolalitätsregulatoren, Antioxidantien, Chelatbildner, Desinfektionsmittel, Dispergiermittel, Emuisionsstäbilisatoren, Hydrokolloide, Netzmittel oder ein Gemisch aus wenigstens zwei der vorstehend genannten Hilfsstoffe enthält.

17. Verwendung gemäß einem der Ansprüche 14 bis 16, **dadurch gekennzeichnet, daß** die Verbindung(en) gemäß Ansprüchen 1 bis 6 in Form eines Gels oder einer Lösung in einer Konzentration von 0,005 mg/ml bis 200 mg/ml, vorzugsweise von 0,5 mg/ml bis 200 mg/ml, besonders bevorzugt 10 mg/ml bis 150 mg/ml vorliegen.

## Claims

1. Use of at least one of the following compounds:
7-(3-amino-1-pyrrolidinyl)-8-chloro-1-cyclopropyl-6-fluoro-1,4-dihydro-4-oxo-3-quinolinecarboxylic acid (clinafloxacin), 1-cyclopropyl-6-fluoro-1,4-dihydro-8-methoxy-7-(3-methyl-1-piperazinyl)-4-oxo-3-quinolinecarboxylic acid (gatifloxacin), 7-[(4Z)-3-(aminomethyl)-4-(methoxyimino)-1-pyrrolidinyl]-1-cyclopropyl-6-fluoro-1,4-dihydro-4-oxo-1,8-naphthyridine-3-carboxylic acid (gemifloxacin), 1-cyclopropyl-6-fluoro-1,4-dihydro-5-methyl-7-(3-methyl-1-piperazinyl)-4-oxo-3-quinolinecarboxylic acid (grepafloxacin), (3S)-9-fluoro-2,3-dihydro-3-methyl-10-(4-methyl-1-piperazinyl)-7-oxo-7H-pyrido[1,2,3-de]-1,4-benzoxazine-6-carboxylic acid (levofloxacin), 1-cyclopropyl-6-fluoro-1,4-dihydro-8-methoxy-7-[(4aS,7aS)-octahydro-6H-pyrrolo[3,4-b]pyridin-6-yl]-4-oxo-3-quinolinecarboxylic acid (moxifloxacin), 5-amino-1-cyclopropyl-7-[(3R,5S)-3,5-dimethyl-1-piperazinyl]-6,8-difluoro-1,4-dihydro-4-oxo-3-quinolinecarboxylic acid (sparfloxacin), 7-(3-amino-1-pyrrolidinyl)-1-(2,4-difluorophenyl)-6-fluoro-1,4-dihydro-4-oxo-1,8-naphthyridine-3-carboxylic acid (tosufloxacin) and 7-(1α,5α,6α-6-amino-3-azabicyclo[3.1.0]hex-3-yl)-1-(2,4-difluorophenyl)-6-fluoro-1,4-dihydro-4-oxo-1,8-naphthyridine-3-carboxylic acid (trovafloxacin) and/or at least one compound of general formula (II): 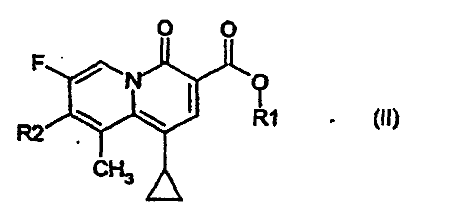 in which:
R1 is hydrogen or C₁-C₃-alkyl, and R2 is an optionally monosubstituted or polysubstituted mono-, bi- or tricyclic alicycle which is saturated or has at least one double bond and which optionally has at least one heteroatom in the ring system, or an aromatic mono-, bi- or tricycle optionally having at least one heteroatom,
and/or its corresponding hydrate and/or its corresponding physiologically compatible acid addition salt and/or optionally its corresponding physiologically compatible salt of the carboxylic acid on which the compounds enumerated are based and/or of the parent carboxylic acid for compounds of general formula (II), in which R1 is H, and/or of corresponding enantiomers and/or corresponding diastereomers and/or of the corresponding racemate and/or of a corresponding mixture of at least two of the abovementioned compounds,
and optionally other physiologically compatible auxiliary substances, for the preparation of a medicament for the topical and/or local treatment and/or for the prophylaxis of diseases caused by bacteria in humans or animals in the region of the mouth and/or teeth and/or jaws.

2. Use according to Claim 1, **characterized in that**, in general formula (II):
R1 is hydrogen, and
R2 is optionally monosubstituted or poly-substituted cyclopropyl, azetidinyl, pyrrolidinyl, piperidinyl, piperazinyl or morpholinyl, it optionally also being possible for at least two substituents to be coupled together.

3. Use according to Claim 1 or 2, **characterized in that** 7-(3-amino-1-pyrrolidinyl)-8-chloro-1-cyclopropyl-6-fluoro-1,4-dihydro-4-oxo-3-quinolinecarboxylic acid (clinafloxacin), 1-cyclopropyl-6-fluoro-1,4-dihydro-8-methoxy-7-(3-methyl-1-piperazinyl)-4-oxo-3-quinolinecarboxylic acid (gatifloxacin), 7-[(4Z)-3-(aminomethyl)-4-(methoxyimino)-1-pyrrolidinyl]-1-cyclopropyl-6-fluoro-1,4-dihydro-4-oxo-1,8-naphthyridine-3-carboxylic acid (gemifloxacin), 1-cyclopropyl-6-fluoro-1,4-dihydro-8-methoxy-7-[(4aS,7aS)-octahydro-6H-pyrrolo[3,4-b]pyridin-6-yl]-4-oxo-3-quinolinecarboxylic acid (moxifloxacin), 5-amino-1-cyclopropyl-7-[(3R,5S)-3,5-dimethyl-1-piperazinyl]-6,8-difluoro-1,4-dihydro-4-oxo-3-quinolinecarboxylic acid (sparfloxacin), 7-(3-amino-1-pyrrolidinyl)-1-(2,4-difluorophenyl)-6-fluoro-1,4-dihydro-4-oxo-1,8-naphthyridine-3-carboxylic acid (tosufloxacin) and 7-(1α,5α,6α-6-amino-3-azabicyclo[3.1.0]hex-3-yl)-1-(2,4-difluorophenyl)-6-fluoro-1,4-dihydro-4-oxo-1,8-naphthyridine-3-carboxylic acid (trovafloxacin) or a mixture of at least two of these compounds, is used.

4. Use according to one of Claims 1 to 3, **characterized in that** 1-cyclopropyl-6-fluoro-1,4-dihydro-8-methoxy-7-(3-methyl-1-piperazinyl)-4-oxo-3-quinolinecarboxylic acid (gatifloxacin), 7-[(4Z)-3(aminomethyl)-4-(methoxyimino)-1-pyrrolidinyl]-1-cyclopropyl-6-fluoro-1,4-dihydro-4-oxo-1,8-naphthyridine-3-carboxylic acid (gemifloxacin) and 1-cyclopropyl-6-fluoro-1,4-dihydro-8-methoxy-7-[(4aS,7aS)-octahydro-6H-pyrrolo[3,4-b]pyridin-6-yl]-4-oxo-3-quinolinecarboxylic acid (moxifloxacin), or a mixture of at least two of these compounds, is used.

5. Use according to one of Claims 1 to 4, **characterized in that** the acid addition salts are selected from the group consisting of hydrochloride, hydrobromide, methanesulfonate and tolylsulfonate.

6. Use according to one of Claims 1 to 5, **characterized in that** the physiologically compatible salts of the carboxylic acid are selected from the group consisting of alkali metal salts, alkaline earth metal salts, ammonium salts, silver salts and guanidinium salts.

7. Use of at least one of the compounds according to one of Claims 1 to 6 for the preparation of a medicament for the local and/or topical treatment of endodontal and periodontal diseases.

8. Use according to one of Claims 1 to 7, **characterized in that** the medicament is used for the local treatment of pulpitis due to carious diseases, for the topical treatment of the infected root canal and the periapical tissue, for the topical treatment of periodontal disease, for the treatment of osseomucosal wounds with disturbed wound healing or for the treatment of soft tissue infections.

9. Use of at least one of the compounds according to one of Claims 1 to 6 for the preparation of a medicament for the prophylaxis of wounds, preferably dentin wounds, or for perioperative prophylaxis.

10. Use according to Claim 9 for perioperative prophylaxis in the case of implantations, heart operations, transplantations, neurosurgical operations, operations in a highly contaminated area such as the oral cavity, oesophagus, rectum or colon, or in hysterectomy, bile duct operations, operations on patients with lowered resistance, or in the case of amputations.

11. Use of at least one of the compounds according to one of Claims 1 to 6 for the preparation of a medicament for the topical and/or local treatment of diabetic foot syndrome.

12. Use of at least one of the compounds according to one of Claims 1 to 6 for the preparation of a medicament for the acceleration of wound healing.

13. Use according to one of Claims 7 to 12, **characterized in that** the medicament is used for the treatment of humans or animals.

14. Use according to one of Claims 1 to 13, **characterized in that** the medicament is in the form of a gel, a solution, a suspension, an emulsion, liposomes or micelles and has optionally been applied to or incorporated in a carrier material or an inert carrier , preferably a strip insert, thread insert, chip, tray, collagen sponge, tampon, cotton wool plug or foam pellet.

15. Use according to Claim 14, **characterized in that** the medicament is in the form of an aqueous solution.

16. Use according to Claim 14 or 15, **characterized in that** the medicament contains, as other physiologically compatible auxiliary substances, solvents, gel formers, solubility improvers, thickeners, solubilizers, preservatives, emulsifiers, mucins, osmolality regulators, antioxidants, chelating agents, disinfectants, dispersants, emulsion stabilizers, hydrocolloids, wetting agents or a mixture of at least two of the abovementioned auxiliary substances.

17. Use according to one of Claims 14 to 16, **characterized in that** the compound(s) according to Claims 1 to 6 are present in the form of a gel or a solution in a concentration of 0.005 mg/ml to 200 mg/ml, preferably 0.5 mg/ml to 200 mg/ml, more preferably 10 mg/ml to 150 mg/ml.

## Revendications

1. Utilisation d'au moins un des composés :
acide 7-(3-amino-1-pyrrolidinyl)-8-chloro-1-cyclopropyl-6-fluoro-1,4-dihydro-4-oxo-3-quinoléinecarboxylique (clinafloxacine), acide 1-cyclopropyl-5-fluoro-1,4-dihydro-8-méthoxy-7-(3-méthyl-1-pipérazinyl)-4-oxo-3-quinoléinecarboxylique (gatifloxacine), acide 7-[(4Z)-3-(aminométhyl)-4-(méthoxyimino)-1-pyrrolidinyl]-1-cyclopropyl-6-fluoro-1,4-dihydro-4-oxo-1,8-naphthyridine-3-carboxylique (gémifloxacine), acide 1-cyclopropyl-6-fluoro-1,4-dihydro-5-méthyl-7-(3-méthyl-1-pipérazinyl)-4-oxo-3-quinoléinecarboxylique (grépafloxacine), acide (3S)-9-fluoro-2,3-dihydro-3-méthyl-10-(4-méthyl-1-pipérazinyl)-7-oxo-7H-pyrido[1,2,3-de]-1,4-benzoxazine-6-carboxylique (lévofloxacine), acide 1-cyclopropyl-6-fluoro-1,4-dihydro-8-méthoxy-7-[(4aS,7aS)-octahydro-6H-pyrrolo[3,4-b]pyridin-6-yl]-4-oxo-3-quinoléinecarboxylique (moxifloxacine), acide 5-amino-1-cyclopropyl-7-[(3R,5S)-3,5-diméthyl-1-pipérazinyl]-6,8-difluoro-1,4-dihydro-4-oxo-3-quinoléinecarboxylique (sparfloxacine), acide 7-(3-amino-1-pyrrolidinyl)-1-(2,4-difluorophényl)-6-fluoro-1,4-dihydro-4-oxo-1,8-naphthyridine-3-carboxylique (tosufloxacine) et acide 7-(1α,5α,6α-6-amino-3-azabicyclo[3.1.0]hex-3-yl)-1-(2,4-difluorophényl)-6-fluoro-1,4-dihydro-4-oxo-1,8-naphthyridine-3-carboxylique (trovafloxacine) et/ou au moins un composé de la formule générale (II) 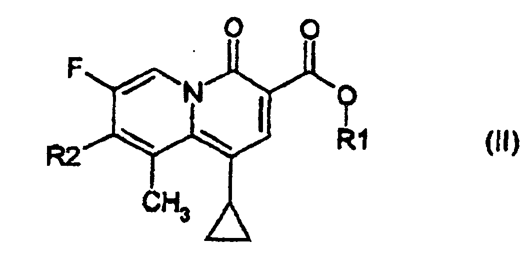
dans laquelle
R1 représente l'hydrogène ou un résidu C₁-C₃-alkyle et
R2 représente un cycle aliphatique mono-, bi- ou tricyclique, à substitution simple ou multiple, le cas échéant, ou présentant au moins une liaison double, ayant, le cas échéant, au moins un hétéroatome saturé dans le système cyclique ou un cycle aromatique mono-, bi- ou tricyclique présentant, le cas échéant, au moins un hétéroatome,
et/ou de son hydrate correspondant et/ou de son sel d'addition d'acide, physiologiquement compatible, correspondant et/ou, le cas échéant, de son sel physiologiquement compatible correspondant de l'acide carboxylique de base des composés énumérés et/ou de l'acide carboxylique de base pour des composés de la formule générale (II), où R1 représente l'hydrogène et/ou des énantiomères correspondants et/ou des diastéréomères correspondants et/ou du mélange racémique correspondant et/ou d'un mélange correspondant d'au moins deux des composés cités ci-avant
ainsi que, le cas échéant, d'autres adjuvants physiologiquement compatibles, en vue de la fabrication d'un médicament destiné au traitement local et/ou topique et/ou à la prophylaxie de maladies d'origine bactérienne dans le domaine buccal et/ou dans le domaine dentaire et/ou dans le domaine des mâchoires chez l'homme et les animaux.

2. Utilisation selon la revendication 1, **caractérisée en ce que**, dans la formule générale (II),
R1 représente l'hydrogène et
R2 représente un résidu cyclopropyle, azétidinyle, pyrrolidinyle, pipéridinyle, pipérazinyle ou morpholinyle, à substitution simple ou multiple, le cas échéant, au moins deux substituants pouvant également être reliés l'un à l'autre le cas échéant.

3. Utilisation selon la revendication 1 ou 2, **caractérisée en ce que** l'on utilise l'acide 7-(3-amino-1-pyrrolidinyl)-8-chloro-1-cyclopropyl-6-fluoro-1,4-dihydro-4-oxo-3-quinoléinecarboxylique (clinafloxacine), l'acide 1-cyclopropyl-6-fluoro-1,4-dihydro-8-méthoxy-7-(3-méthyl-1-pipérazinyl)-4-oxo-3-quinoléinecarboxylique (gatifloxacine), l'acide 7-[(4Z)-3-(aminométhyl)-4-(méthoxyimino)-1-pyrrolidinyl]-1-cyclopropyl-6-fluoro-1,4-dihydro-4-oxo-1,8-naphthyridine-3-carboxylique (gémifloxacine), l'acide 1-cyclopropyl-6-fluoro-1,4-dihydro-8-méthoxy-7-[(4aS,7aS)-octahydro-6H-pyrrolo[3,4-b]pyridin-6-yl]-4-oxo-3-quinoléinecarboxylique (moxifloxacine), l'acide 5-amino-1-cyclopropyl-7-[(3R,5S)-3,5-diméthyl-1-pipérazinyl]-6,8-difluoro-1,4-dihydro-4-oxo-3-quinoléinecarboxylique (sparfloxacine), l'acide 7-(3-amino-1-pyrrolidinyl)-1-(2,4-difluorophényl)-6-fluoro-1,4-dihydro-4-oxo-1,8-naphthyridine-3-carboxylique (tosufloxacine) et l'acide 7-(1α,5α,6α-6-amino-3-azabicyclo[3.1.0]hex-3-yl)-1-(2,4-difluorophényl)-6-fluoro-1,4-dihydro-4-oxo-1,8-naphthyridine-3-carboxylique (trovafloxacine) ou un mélange d'au moins deux de ces composés.

4. Utilisation selon l'une quelconque des revendications 1 à 3, **caractérisée en ce que** l'on utilise l'acide 1-cyclopropyl-6-fluoro-1,4-dihydro-8-méthoxy-7-(3-méthyl-1-pipérazinyl)-4-oxo-3-quinoléinecarboxylique (gatifloxacine), l'acide 7-[(4Z)-3-(aminométhyl)-4-(méthoxyimino)-1-pyrrolidinyl]-1-cyclopropyl-6-fluoro-1,4-dihydro-4-oxo-1,8-naphthyridine-3-carboxylique (gémifloxacine) et l'acide 1-cyclopropyl-6-fluoro-1,4-dihydro-8-méthoxy-7-[(4aS,7aS)-octahydro-6H-pyrrolo[3,4-b]pyridin-6-yl]-4-oxo-3-quinoléinecarboxylique (moxifloxacine) ou un mélange d'au moins deux de ces composés.

5. Utilisation selon l'une quelconque des revendications 1 à 4, **caractérisé en ce que** les sels d'addition d'acide sont sélectionnés parmi le groupe hydrochlorure, hydrobromure, méthanesulfonate et tolylsulfonate.

6. Utilisation selon l'une quelconque des revendications 1 à 5, **caractérisée en ce que** les sels physiologiquement compatibles de l'acide carboxylique sont choisis parmi le groupe des sels alcalins, des sels alcalino-terreux, des sels d'ammonium, des sels d'argent et des sels du guanidinium.

7. Utilisation d'au moins un des composés selon l'une quelconque des revendications 1 à 6 en vue de la fabrication d'un médicament destiné au traitement local et/ou topique de maladies endodontiques et parodontiques.

8. Utilisation selon l'une quelconque des revendications 1 à 7, **caractérisée en ce que** l'on utilise le médicament destiné au traitement local de pulpites provenant de maladies carieuses, au traitement topique du canal à racine infecté et du tissu périapical, au traitement topique de la parodontopathie, au traitement de lésions osseuses des muqueuses à guérison perturbée ou au traitement d'infections de tissus mous.

9. Utilisation d'au moins un des composés selon l'une quelconque des revendications 1 à 6 en vue de la fabrication d'un médicament pour la prophylaxie de lésions, de préférence de lésions dentaires ou en vue de la prophylaxie périopératoire.

10. Utilisation selon la revendication 9 en vue de prophylaxie périopératoire lors d'implantations, d'opérations cardiaques, de transplantations, d'opérations neurochirurgicales, d'opérations dans des domaines fortement contaminés comme la cavité buccale, l'oesophage, le rectum ou le colon ou en cas d'hystérectomie, d'opérations des canaux biliaires, en cas d'opérations de patients ayant une immunodéficience ou lors d'amputations.

11. Utilisation d'au moins un des composés selon l'une quelconque des revendications 1 à 6 en vue de la fabrication d'un médicament pour le traitement topique et/ou local du syndrome du pied diabétique.

12. Utilisation d'au moins un des composés selon l'une quelconque des revendications 1 à 6 en vue de la fabrication d'un médicament pour l'accélération de la guérison des blessures.

13. Utilisation selon l'une quelconque des revendications 7 à 12, **caractérisée en ce que** le médicament est utilisé en vue du traitement de l'homme ou des animaux.

14. Utilisation selon l'une quelconque des revendications 1 à 13, **caractérisée en ce que** le médicament se présente sous la forme d'un gel, d'une solution, d'une suspension, d'une émulsion, sous la forme de liposomes ou sous la forme de micelles et est appliqué sur ou introduit dans, le cas échéant, un matériau vecteur ou un vecteur inerte, de préférence une garniture en bande, une garniture en fils, une puce, une plaquette, une éponge de collagène, un tampon, un tampon d'ouate, une pastille en mousse.

15. Utilisation selon la revendication 14, **caractérisée en ce que** le médicament se présente sous la forme d'une solution aqueuse.

16. Utilisation selon la revendication 14 ou 15, **caractérisée en ce que** le médicament contient, en tant qu'autres adjuvants physiologiquement compatibles, des solvants, des agents gélifiants, des agents d'amélioration de la solubilité, des agents épaississants, des diluants, des agents de solubilisation, des agents de conservation, des émulsifiants, des substances visqueuses, des agents régulateurs de l'osmolalité, des agents anti-oxydants, des agents chélateurs, des désinfectants, des agents de dispersion, des agents de stabilisation d'émulsions, des hydrocolloïdes, des agents mouillants ou un mélange d'au moins deux des adjuvants suscités.

17. Utilisation selon l'une quelconque des revendications 14 à 16, **caractérisée en ce que** le ou les composés selon les revendications 1 à 6 se présentent sous la forme d'un gel ou d'une solution, dans une concentration de 0,005 mg/ml à 200 mg/ml, de préférence de 0,5 mg/ml à 200 mg/ml, tout particulièrement de préférence de 10 mg/ml à 150 mg/ml.
